# EUROPEAN PATENT SPECIFICATION

(11) **EP 3 502 269 B1**
(45) Date of publication and mention of the grant of the patent: **24.02.2021**
(21) Application number: 18209939.0
(22) Date of filing: 09.07.2014
(51) Int. Cl.: C12Q 1/68

(54) **DETECTION OF BRAIN CANCER**
NACHWEIS VON HIRNKREBS
DÉTECTION DU CANCER DU CERVEAU

(30) Priority: 09.07.2013 GB 201312336; 10.01.2014 GB 201400436
(43) Date of publication of application: 26.06.2019
(62) Divisional of application: 14739904.2
(73) Proprietor: University of Central Lancashire, Preston, Lancashire PR1 2HE (GB)
(72) Inventor: SHAW, Lisa, Preston Lancashire PR1 2HE (GB); TUMILSON, Charlotte, Rolleston 7615 (NZ); ALDER, Jane, Preston Lancashire PR1 2HE (GB)
(74) Representative: HGF

(56) References cited:
- WO-A1-2012/114189
- WO-A2-2012/089753
- US-A1- 2012 322 680
- Patrick Roth ET AL: "A specific miRNA signature in the peripheral blood of glioblastoma patients", Journal of Neurochemistry, vol. 118, no. 3, 1 August 2011 (2011-08-01), pages 449-457, XP055209586, ISSN: 0022-3042, DOI: 10.1111/j.1471-4159.2011.07307.x

## Description

The present invention relates to methods for detecting glioma in a subject. The invention also relates to methods of predicting a clinical outcome in a patient with glioma and methods of monitoring the progression of glioma in a patient.

### INTRODUCTION

In 2010, there were 9,156 new cases of brain cancer in the UK alone (Cancer Research UK). Worldwide it is estimated that there are 445,000 new cases of brain cancer every year (Cancer Research UK). As the population grows every year, so the incidence of brain cancer will follow, highlighting the need for improved diagnosis, prognosis and prediction of response to treatment. This invention has the potential to fulfil this need both in the UK and worldwide.

Current diagnosis of glioma involves imaging with MRI and histological analysis by neuropathology. In some cases MRI scans are not accurate enough to definitively diagnose an individual, following this histological analysis is required. Histological analysis can only be performed following removal of tumour tissue during surgery and it is extremely subjective depending on the interpretations of individual pathologists. A risky and invasive procedure for patients, especially as there is a high incidence of glioma in older individuals who present a higher risk when undergoing surgery.

MicroRNAs (miRNA) are small non-coding RNAs which play a role in post-transcriptional regulation of gene and protein expression. MiRNAs exhibit disease specific expression, which can be used to provide information about a particular biological state, such as glioma. Changes in miRNA expression in gliomas can be measured following the isolation of glioma specific exosomes released into the circulation. The aim of this study is to identify a panel of miRNAs which have an altered expression in glioma and can be used for diagnosis, prognosis and the prediction of response to treatment.

WO2012/089753 discloses has-miR-20a-5p as a biomarker for glioblastoma. WO2012/114189 discloses a process of determining survival prognosis in a subject with glioblastoma wherein said process comprises (a) measuring the level of microRNAs including has-miR-20a. US2012/322680 discloses in vitro diagnosis of a brain tumour such as glioblastoma by measuring the expression level of has-miR-20a. Roth et al. 'A specific miRNA signature in the peripheral blood of glioblastoma patients' Journal of Neurochemistry vol.118 no.3, August 2011 describes a set of blood derived miRNA biomarkers for glioma.

In a first aspect, the invention provides a method of detecting glioma in a subject, the method comprising:
assaying a serum sample from the subject to determine the amount of at least one miRNA, selected from the group consisting of: Hsa-miR-20a-5p, present in the sample;
comparing the amount of the at least one miRNA present in the serum sample with a reference value;
wherein an increase in the amount of at the least one miRNA in the serum sample, as compared to the reference value, indicates that the subject has glioma, and wherein if the increase is such that the amount of the at least one miRNA in the serum sample is more than twice the reference value, this indicates a positive outcome in respect of the patient's glioma..

This first aspect of the present invention is based upon the inventors' finding that the miRNAs referred to above have a change in abundance in samples that are representative of glioma (either being taken from individuals that have gliomas, or from cell culture models of glioma) as compared to subjects without glioma (or models using non-cancerous brain cells). Indeed, as discussed in more detail elsewhere in the present disclosure, the inventors have found that the miRNAs referred to are present in quantities that are multiple times ("fold" increases) higher or lower than those found in non-cancerous reference samples.

In a second aspect of the invention, there is provided a method of predicting a clinical outcome in a patient with glioma, the method comprising:
assaying a serum sample from the patient for the presence of at least one miRNA selected from the group consisting of: Hsa-miR-20a-5p; comparing the amount of the at least one miRNA present in the serum sample with a reference value;
wherein an increase in the amount of at the least one miRNA in the serum sample, such that the amount is more than twice the reference value indicates a positive outcome in respect of the patient's glioma, wherein the reference value is the level of the same at least one miRNA present in a control subject without cancer.

In one embodiment of the second aspect, a decrease in the amount of the at least one miRNA in the serum sample, as compared to twice the reference value, indicates a negative outcome in respect of the patient's glioma.

This second aspect of the present invention is based upon the inventors' finding of the link between the miRNAs referred to and the presence of glioma. This finding allows the investigation of the miRNAs to above provide an indication as to the likely progression of a patient's glioma, and accordingly the clinical outcome that may be expected. In particular, the inventors have found that changes in the abundance of these miRNA markers are generally associated with a negative outcome in respect of the patient's brain cancer.

Techniques for use in the diagnosis, prognosis and monitoring of brain cancers currently typically use an approach of imaging, such as MRI techniques, followed by confirmatory histological analysis.

It is also disclosed herein that the panel of miRNA biomarkers identified herein may be of benefit in the grading of brain cancers such as gliomas.

In a third aspect the invention provides a method of monitoring the progression of glioma in a patient, the method comprising:
assaying a first serum sample from the patient, indicative of miRNA in the patient at a first timepoint, for the presence of at least one miRNA selected from the group consisting of: hsa-Hsa-miR-20a-5p; to determine a first value for the abundance of the at least one miRNA present in the serum sample;
and assaying a second serum sample from the patient, indicative of miRNA in the patient at a second timepoint, for the presence of the same at least one miRNA to determine a second value for the abundance of the at least one miRNA present in the second serum sample; and comparing the first and second values for the abundance of the at least one miRNA;
wherein an increase in the amount of the at least miRNA present in the second serum sample as compared to the first serum sample, such that the amount is more than twice the level of the same at least one miRNA present in a control subject without cancer, indicates an improvement in respect of the patient's brain cancer.

In one embodiment of the third aspect, a decrease in the amount of the at least one miRNA present in the second serum sample as compared to the first serum sample, such that the amount is less than twee the level of the same at least one miRNA present in a control subject without cancer, indicates a worsening in respect of the patient's glioma.

When the miRNA investigated is one that is up-regulated in cancer as compared to reference values, then an increase in the second value as compared to the first value may indicate that the progression of the brain cancer has been to worsen between the first and second timepoints.

When the miRNA investigated is one that is down-regulated in cancer as compared to reference values, then decrease in the second value as compared to the first value may also indicates that the progression of the brain cancer has been to worsen between the first and second timepoints.

In contrast, when the miRNA investigated is one that is up-regulated in cancer as compared to reference values, then a decrease in the second value as compared to the first value may indicate that the progression of the brain cancer has been to improve between the first and second timepoints.

Similarly, when the miRNA investigated is one that is down-regulated in cancer as compared to reference values, then an increase in the second value as compared to the first value may indicate that the progression of the brain cancer has been to improve between the first and second timepoints.

The monitoring methods described herein may make use of further samples representative of miRNA in the patient at further timepoints (for example a third sample representative of miRNA in the patient at a third timepoint, in addition to the first and second samples).

The first, second (and any subsequent) timepoints may be separated by any period of interest during which it is wished to monitor progression of the patient's brain cancer.

The patient may be subject to treatment that is intended to alter the status of the cancer between the first and second timepoints. For example, the patient may be subject to clinical treatment, or treatment with a putative therapeutic agent. In this case the monitoring of the progression of the patient's cancer may provide an indication as to whether the clinical treatment, or putative therapeutic agent, is proving successful.

Monitoring of cancer progression in this manner may also be beneficial in determining when cancer treatment should be initiated. Merely by way of example, the initiation of treatment may be indicated when a cancer worsens (e.g. a low grade tumour progressing to a high grade tumour). Similarly, a decision as to whether to begin or end treatment regimens, such as chemotherapy regimens, may be taken with a view to monitored advancement and/or regression of the tumour.

It will be appreciated that multiple miRNAs from the recited lists herein may be assayed for in respect of the two samples, and that different miRNAs may be assayed for, so long as at least one of the miRNAs assayed for is in common between the two samples (thus allowing a comparison to be performed in respect of the abundance of that miRNA in the samples).

Also disclosed herein, there is provided a method of grading a patient's brain cancer, the method comprising:
assaying a sample from the patient to determine the amount of at least one miRNA, selected from the group consisting of: Hsa-miR-20a-5p; Hsa-miR-34a-5p; Hsa-miR-92a-3p hsa-miR-15b; hsa-miR-181b; hsa-miR-19a; hsa-miR-210; hsa-miR-23a; hsa-miR-15a; hsa-miR-181a; hsa-miR-21; hsa-miR-222; hsa-miR-26a; hsa-miR-29c; hsa-miR-101-3p; hsa-miR-29b-3p; hsa-miR-328; hsa-miR-9-5p; Hsa-miR-106b-5p; Hsa-miR-107; Hsa-miR-125a-5p; Hsa-miR-128; Hsa-miR-130b-3p; Hsa-miR-132-3p; Hsa-miR-138-5p; Hsa-miR-141-3p; Hsa-miR-146a-5p; Hsa-miR-148a-3p; Hsa-miR-16-5p; Hsa-miR-17-5p; Hsa-miR-17-3p ; Hsa-miR-182-5p; Hsa-miR-183-5p; Hsa-miR-187-3p; Hsa-miR-18a-5p; Hsa-miR-190a; Hsa-miR-19b-3p; Hsa-miR-200a-3p; Hsa-miR-203a; Hsa-miR-31-5p; Hsa-miR-326; Hsa-miR-425-5p; Hsa-miR-7-5p; Hsa-miR-93-5p; Hsa-miR-96-5p; Hsa-let-7b-5p; Hsa-miR-106b-5p; Hsa-miR-191-5p; Hsa-miR-24-3p; Hsa-miR-25-3p; Hsa-miR-320a; Hsa-miR-486-5p; and Hsa-miR-451a, present in the sample;
comparing the amount of the at least one miRNA present in the sample with reference values for miRNA expression obtained from a cancers of known clinical grades; and
allocating the patient's brain cancer to the clinical grade to which the amount of the at least one miRNA present in the sample most closely resembles.

In one embodiment of the methods of the first, second or third aspects of the invention, the assay used to determine the presence or amount of the at least one miRNA comprises an amplification step in which miRNA in the patient sample is used as a template for the generation of artificial nucleic acid molecules.

The methods of each of the first, second, and third aspects may, for the sake of brevity, be referred to herein as "methods of the invention". The following pages will provide more details of suitable embodiments of these, and other, aspects of the disclosure. Except for where the context requires otherwise embodiments described with reference to one aspect of the invention or disclosure may also be applied to other aspects of the invention or disclosure.

The methods of the invention are able to provide many advantages over those techniques known from the prior art. The method of the first aspect of the invention are able to be used as a diagnostic test for glioma. Through a non-invasive process of blood sampling, serum can be isolated from patients and tested to identify changes in a select panel of microRNAs to determine the presence of a brain tumour without the need for surgery. A simple test when the patient initially presents with neurological symptoms could lead to earlier diagnosis allowing for prompt treatment and giving the patient the best chance at a positive outcome.

Also disclosed herein are methods that can also be used to distinguish between different grades of brain tumour. If such methods are performed at diagnosis; this could be useful in selecting the correct treatment for the patient, again giving them the best chance at a positive outcome without the need for an invasive procedure.

A major benefit of the methods of the invention is that they can be performed without the need for surgery as with histological techniques. They are also able to improve the accuracy of diagnosis, compared to MRI. There is also potential for earlier diagnosis when the tumour may be present but not identifiable by imaging, allowing for prompt treatment and potentially an improved prognosis.

The measurement of the microRNA expression in the serum and/or CSF could overcome the limitations of current diagnostic techniques. By improving accuracy through the analysis of microRNAs but at the same time being non-invasive by the taking of a blood samples rather than surgery.

Analysis of microRNA expression in tumour tissue, when surgery is necessary again, improves accuracy in comparison to current techniques.

The analysis performed by the inventors provides further insight into the manner in which the various biomarker miRNAs herein identified may be used to practice the methods described herein.

A selection of the miRNAs referred to herein show their diagnostic utility when they are up-regulated as compared to reference values. Accordingly, finding an increased abundance of one or more of these diagnostic up-regulated miRNAs in a sample of a subject, who may be of unknown brain cancer status indicates that the subject in question has brain cancer. miRNAs suitable for use in such manner may be selected from the group consisting of: Has-miR-20a-5p; Hsa-miR-34a-5p; Hsa-miR-92a-3p; hsa-miR-101-3p; hsa-miR-148a; hsa-miR-29b-3p; Hsa-let-7b-5p; Hsa-miR-106b-5p; Hsa-miR-24-3p; Hsa-miR-25-3p; Hsa-miR-320a; Hsa-miR-486-5p; Hsa-miR-15b; Hsa-miR-17-5p; Hsa-miR-17-3p; Hsa-miR-181b; Hsa-miR-19a; Hsa-miR-210; and Hsa-miR-23a. Methods described herein may utilise one, more, or all of such miRNAs. Also disclosed is a particularly useful selection of miRNAs that are up-regulated in incidences of cancer and may comprise one or more selected from the group consisting of: Has-miR-20a-5p; Hsa-miR-34a-5p; Hsa-miR-92a-3p; hsa-miR-101-3p; hsa-miR-148a; hsa-miR-29b-3p; Hsa-miR-320a; Hsa-miR-486-5p; Hsa-miR-15b; Hsa-miR-17-5p; Hsa-miR-17-3p; Hsa-miR-181b; Hsa-miR-19a; Hsa-miR-210; and Hsa-miR-23a.

In contrast to the up-regulated miRNA biomarkers referred to above, also disclosed herein are a panel of markers that are down-regulated in cancerous cells as compared to reference values. Accordingly, finding decreased abundance of one or more of these diagnostic down-regulated miRNAs in a sample from a subject, who may otherwise be of unknown brain cancer status, indicates that the subject in question has brain cancer. Thus, an alternative method of the present disclosure may involve assaying a sample from a subject to determine the amount of at least one miRNA selected from the group consisting of: Hsa-miR-191-5p; Hsa-miR-451a; hsa-miR-328; hsa-miR-9-5p; Hsa-miR-106b-5p; Hsa-miR-107; Hsa-miR-125a-5p; Hsa-miR-128; Hsa-miR-130b-3p; Hsa-miR-132-3p; Hsa-miR-138-5p; Hsa-miR-141-3p; Hsa-miR-146a-5p; Hsa-miR-148a-3p; Hsa-miR-16-5p; ; Hsa-miR-182-5p; Hsa-miR-183-5p; Hsa-miR-187-3p; Hsa-miR-18a-5p; Hsa-miR-190a; Hsa-miR-19b-3p; Hsa-miR-200a-3p; Hsa-miR-203a; Hsa-miR-31-5p; Hsa-miR-326; Hsa-miR-425-5p; Hsa-miR-7-5p; Hsa-miR-93-5p; Hsa-miR-15a; Hsa-miR-181a; Hsa-miR-21; Hsa-miR-222; Hsa-miR-26a; Hsa-miR- Hsa-miR-29c; and Hsa-miR-96-5p; and comparing the amount found in the sample to a reference value, wherein a decrease of the amount present in the sample as compared to the reference value indicates the presence of cancer. Methods described herein may utilise one, more, or all of such miRNAs. A particularly useful selection of these miRNAs down-regulated in incidences of cancer may comprise one or more selected from the group consisting of: Hsa-miR-191-5p; Hsa-miR-451a; hsa-miR-328; hsa-miR-9-5p; Hsa-miR-138-5p; Hsa-miR-16-5p; Hsa-miR-19b-3p; Hsa-miR-15a; Hsa-miR-181a; Hsa-miR-21; Hsa-miR-222; Hsa-miR-26a; Hsa-miR-29c; and Hsa-miR-93.

As referred to above, the methods described herein are useful in the prediction of a clinical outcome in a patient with brain cancer. These methods are able to provide an indication as to the patient's expected length of survival with the brain cancer.

Disclosed herein the at least one miRNA may be selected from the group consisting of: hsa-miR-101-3p; hsa-miR-29b-3p; hsa-miR-328; hsa-miR-9-5p; Hsa-let-7b-5p; Hsa-miR-106b-5p; Hsa-miR-191-5p; Hsa-miR-24-3p; Hsa-miR-25-3p; Hsa-miR-320a; Hsa-miR-486-5p; Hsa-miR-451a; Hsa-miR-34a-5p; and Hsa-miR-92a-3p, wherein an increase in the amount of the at least one miRNA present in the sample as compared to the reference value indicates a negative outcome in respect of the patient's brain cancer.

Alternatively disclosed herein, the at least one miRNA may be selected from the group consisting of: Hsa-miR-106b-5p; Hsa-miR-107; Hsa-miR-125a-5p; Hsa-miR-128; Hsa-miR-130b-3p; Hsa-miR-132-3p; Hsa-miR-138-5p; Hsa-miR-141-3p; Hsa-miR-146a-5p; Hsa-miR-148a-3p; Hsa-miR-16-5p;; Hsa-miR-182-5p; Hsa-miR-183-5p; Hsa-miR-187-3p; Hsa-miR-18a-5p; Hsa-miR-190a; Hsa-miR-19b-3p; Hsa-miR-200a-3p; Hsa-miR-203a; Hsa-miR-20a-5p; Hsa-miR-31-5p; Hsa-miR-326; Hsa-miR-425-5p; Hsa-miR-7-5p; Hsa-miR-93-5p; and Hsa-miR-96-5p; and a decrease in the amount of the at least one miRNA present in the sample as compared to the reference value indicates a negative outcome in respect of the patient's brain cancer.

As discussed further below, in the case of a method of the preceding paragraph in which the amount of Hsa-miR-20a-5p present is assessed, a suitable reference value may be a twofold increase as compared to a suitable control (for example a sex and age matched control).

In the case of those microRNAs found to be up-regulated in serum of cancer patients, it may generally be expected that the greater the elevation of regulation, the worse the prognosis for the patient. Similarly, it may be expected that in cases of microRNAs down-regulated in serum of patients with cancer, the more down-regulated the microRNA, the worse the likelihood of clinical outcome for the patient. However, the inventors have found a group of microRNAs that constitute useful indicators of clinical outcome, but which do not follow the pattern set out above.

Hsa-miR-20a-5p; Hsa-miR-92a-3p; and Hsa-miR-34a-5p, are all up-regulated in serum patients with glioma brain cancer, as compared to controls. Surprisingly, the inventors have found that, when the levels of these miRNAs are compared to suitable controls, a higher level of up-regulation is associated with better clinical outcome.

Thus also disclosed herein there is a method which involves assaying a sample from the patient for at least one miRNA selected from the group consisting of: Hsa-miR-20a-5p; Hsa-miR-92a-3p; and Hsa-miR-34a-5p,
comparing the amount of the at least one miRNA present in the sample with a reference value reflecting the level of the same at least one miRNA present in a control subject without cancer;
wherein an decrease in the amount of at the least one miRNA in the sample, as compared to the reference value, indicates a negative outcome in respect of the patient's brain cancer.

In the case of such a method utilising Hsa-miR-20a-5p, a suitable reference value reflecting the level of this miRNA in a control subject without cancer may be a value twice the level of this miRNA in a control subject without cancer. A patient in whom the level of Hsa-miR-20a-5p is greater than the value twice the level in a control subject will be expected to have a positive outcome in respect of their cancer. A patient in whom the level of Hsa-miR-20a-5p is elevated as compared to the level in a control subject, but is not greater than twice the value in a control subject, will be expected to have a negative outcome in respect of their cancer.

In the case of such methods utilising Hsa-miR-34a-5p and/or Hsa-miR-92a-3p, a suitable reference value reflecting the level of this miRNA in a control subject without cancer may be a value twice, three times, or more the level of the miRNA in question in a control subject without cancer. A patient in whom the level of Hsa-miR-34a-5p and/or Hsa-miR-92a-3p is greater than the selected value in a control subject will be expected to have a positive outcome in respect of their cancer. A patient in whom the level of Hsa-miR-34a-5p and/or Hsa-miR-92a-3p is elevated as compared to the level in a control subject, but is not greater than the selected value in a control subject, will be expected to have a negative outcome in respect of their cancer.

Suitably the sample from the patient is a body fluid sample, such as a serum sample.

As disclosed herein, the methods of the invention may further comprise steps involving the selection, and optionally implementation, of an appropriate therapeutic regimen. Thus, for example, a method of detecting brain cancer may further comprise selecting an appropriate treatment regimen for a subject identified as having brain cancer, and optionally providing said treatment regimen.

In the case of methods for predicting a clinical outcome in a patient with brain cancer, the method may further comprise selecting an appropriate treatment regimen for a subject identified as at risk of a negative outcome in respect of their brain cancer, and may optionally further involve providing said treatment regimen. Suitable treatments in this context may include more radical treatments than those that would be deemed clinically appropriate in respect of a patient viewed as likely to have a positive clinical outcome.

In the case of methods for monitoring the progression of brain cancer in a patient, the method may further comprise making the decision to initiate treatment for brain cancer in the event that the method indicates a worsening in respect of the patient's brain cancer. The method may optionally comprise the initiation of the selected treatment. If the patient is already undergoing treatment, but the method indicates that the patient's brain cancer is still worsening, then the further step may involve a decision to select a more radical treatment regimen, and optionally the provision of the selected more radical treatment regimen. In the event that a method indicates an improvement in respect of the patient's brain cancer, a suitable further step of the method may involve reducing or ceasing any ongoing treatment.

In the case of methods in which a patient's brain cancer is graded, the method may further comprise selecting, and optionally providing, a treatment regimen appropriate to the clinical grade that has been allocated to the patient's brain cancer.

Suitable treatments for brain cancer, that may be provided as further steps of the methods of the disclosure, including more or less radical treatments for brain cancer, will be known to those skilled in the art.

For the avoidance of doubt, and in order to clarify the way in which the present disclosure is to be interpreted, certain terms used in accordance with the present invention will now be defined further.

### Samples suitable for use in the methods of the invention

The methods of the invention make use of samples that provide useful biological information regarding the presence of miRNA biomarkers within the subject undergoing investigation.

### i) Tissue samples

As disclosed herein, the sample may be a tissue sample. Merely by way of example, the sample may be a biopsy sample, such as a brain biopsy sample.

Although the collection of tissue samples, such as biopsy samples, is generally more invasive than the collection of body fluid samples (discussed below), it may still represent a commonly used procedure in many clinical contexts. In such cases the tissue sample from the subject may suitably be assayed to determine the amount of at least one miRNA of interest in the sample.

Any of the down-regulated miRNAs discussed above may be used in methods practiced on tissue samples.

While the inventors believe that all of the biomarker miRNAs referred to herein have diagnostic utility when down-regulated in a sample (as compared to a reference value) certain of these markers show particularly marked changes in their abundance. Thus, disclosed herein is a method involving comparing the level of at least one miRNA selected from the group consisting of Hsa-miR-141-3p; Hsa-miR-148a-3p; Hsa-miR-16-5p; Hsa-miR-182-5p; Hsa-miR-18a-5p; Hsa-miR-200a-3p; Hsa-miR-203a; Hsa-miR-31-5p; Hsa-miR-326; and Hsa-miR-425-5p with a reference value, wherein a decrease in the amount of said at least one miRNA in the sample, as compared to the reference value, is indicative of cancer.

The inventors have also found that the certain of the up-regulated miRNAs described above are particularly increased in brain cancer cells, and so may be of notable diagnostic utility in methods in which the sample is a tissue sample. Accordingly, the methods disclosed herein may involve assaying a tissue sample for the level of at least one miRNA selected from the group consisting of: hsa-miR-101-3p; hsa-miR-29b-3p; hsa-miR-328; and hsa-miR-9-5p, wherein increased abundance of the at least one selected miRNA in said sample, as compared to a reference value, is indicative of the presence of cancer.

### ii) Body fluid samples

Alternatively, the sample is a body fluid sample. In the present disclosure, the body fluid sample may be selected from the group consisting of: a cerebrospinal fluid (CSF) sample; a blood sample; and a serum sample. In accordance with the invention, the sample is a serum sample.

The skilled person will appreciate that under normal circumstances it can be difficult to obtain detailed information regarding biological processes, such as the presence or progression of cancer, occurring within the brain. The brain is separated from much of the body by the blood brain barrier, and is physically enclosed and protected within the skull. These considerations, and the difficulties that they impose upon the diagnosis or monitoring of brain cancer, have already been discussed elsewhere in the specification.

In light of these known difficulties, it will be appreciated that body fluid samples may represent particularly useful examples of samples that can be used in the methods disclosed herein since they may generally be obtained by less invasive procedures than tend to be necessary in order to obtain tissue samples, such as biopsy samples, from the brain. In particular, samples such as serum samples are especially easy and safe to obtain, since they merely the taking of a blood sample. It is both highly advantageous and surprising that the methods of the invention are able to provide information, such as about the presence or progression of cancer in the brain, using serum which (as a constituent of blood) is usually separated from the brain by the blood brain barrier.

The use of biological fluids as a means of monitoring the diagnosis of the disease state and prognosis is a relatively non-invasive procedure. It circumvents the need for risky surgery and offers a definitive diagnosis thus eliminating subjective interpretation in histopathological sections.

For individuals which surgery is necessary, microRNA expression of tumour tissue again provides improved accuracy for diagnosis, prognostic information and predicting response to treatment.

In methods disclosed herein in which the sample is bodily fluid, such as a serum sample, the presence of cancer may be indicated by an increase in the abundance of at least one miRNA selected from the group consisting of: Hsa-let-7b-5p; Hsa-miR-106b-5p; Hsa-miR-191-5p; Hsa-miR-24-3p; Hsa-miR-25-3p; Hsa-miR-320a; Hsa-miR-486-5p; Hsa-miR-451a; Hsa-miR-34a-5p; and Hsa-miR-92a-3p;as compared to a reference value.

In methods disclosed herein in which the sample is a serum sample, and the subject is male, the presence of cancer may be indicated by an increase in the abundance of at least one miRNA selected from the group consisting of: Hsa-let-7b-5p; Hsa-miR-106b-5p; Hsa-miR-191-5p; Hsa-miR-24-3p; Hsa-miR-25-3p; Hsa-miR-320a; and Hsa-miR-486-5p as compared to a reference value.

Disclosed herein is a method in which the sample is a serum sample, and the subject is male, the inventors have found that a subset of the miRNA markers referred to above undergo particularly marked increases in abundance. Accordingly, in such a method the presence of cancer may be indicated by an increase in the abundance of at least one miRNA selected from the group consisting of: Hsa-miR-191-5p; Hsa-miR-486; Hsa-let-7b-5p; and Hsa-miR-25-3p as compared to a reference value. The inventors consider these markers to have particular diagnostic value in such methods of the disclosure.

Disclosed is a method in which the sample is a serum sample, and the subject is female, the presence of cancer may then be indicated by an increase in the abundance of at least one miRNA selected from the group consisting of: Hsa-miR-451a; Hsa-miR-486-5p; Hsa-miR-92a-3p; and Hsa-miR-25-3p as compared to a reference value.

Also disclosed herein are methods using serum samples from female subjects, the presence of cancer may be indicated by an increase in the abundance of at least one miRNA selected from the group consisting of: Hsa-miR-486-5p; and Hsa-miR-25-3p as compared to a reference value. These miRNAs represent biomarkers that are particularly strongly up-regulated in the serum of female subjects with brain cancer, and so are considered to be of notable diagnostic utility.

Also disclosed herein are methods in which the sample is a serum sample, and the subject is aged 60 years, or over, the presence of cancer may then be indicated by an increase in the abundance of Hsa-miR-34a-5p as compared to control values. Suitable controls may be appropriately age-matched. The inventors have found that Hsa-miR-34a is a biomarker that is up-regulated in the serum of subjects aged 60 or over with brain cancer, and so Hsa-miR-34a-5p is considered to be of particular diagnostic utility in serum samples from individuals of this age.

### "Comparing"

The step of comparing the amounts of the miRNAs in a sample with those in a reference value will generally merely require that sufficient information be available to determine whether the abundance of the miRNA present in the sample is increased or decreased as compared to the reference value. As disclosed herein, the information available may be sufficient to allow the identification of fold-changes in the abundance of the miRNA as compared to the reference value.

### "Difference" between amounts of miRNA in a sample and the reference value

The difference between the amount of the miRNA present in the sample and the reference value may be either a relative increase in the abundance of the miRNA in the sample as compared to the reference value, or a relative decrease in the abundance of the miRNA in the sample as compared to the reference value. The nature of the "difference" that is relevant to the use of different miRNA markers (e.g. whether a particular marker has diagnostic utility if increased or decreased as compared to a reference valued) is discussed elsewhere in the present disclosure

### "Reference value"

The methods described herein make use of comparison between the amount of a miRNA of interest that is present in a sample, and a suitable reference value. This comparison with the reference value allows an assessment to be made as to whether the abundance of the miRNA in question is increased or decreased as compared to a suitable control.

It will be appreciated that a reference value may be determined by parallel processing of a suitable control sample in the same manner as the sample of interest. However this need not be the case, and the methods of the disclosure can be practice making use of standardised information as to the levels of the miRNAs of interest in suitable control samples.

Some of these miRNAs of interest are secreted, while others are not. This distinction allows sub-selections of miRNAs to be detected with reference to the type of sample available.

The recognition that certain of the miRNAs that may be utilised in the methods of the disclosure are secreted also has a significant impact upon the selection of appropriate reference values. If a miRNA indicative of brain cancer is secreted into body fluids then suitable reference values must be determined from subjects known not to have cancer, since otherwise there is a risk that at least some contamination from an unknown cancer may occur, even in samples collected from a site distant from the brain cancer.

Similarly, the recognition that certain miRNAs useful in the methods of the disclosure are not secreted also has a significant impact upon the selection of suitable samples and reference values for use in methods in which these miRNAs are to be assessed. In contrast to the secreted miRNAs, these non-secreted miRNAs are not suitable for assessment in body fluid samples. Suitable reference values may be determined from non-cancerous tissue samples. Alternatively, suitable reference values may be determined from cultured cells known to be non-cancerous.

Also disclosed herein is a tissue sample, in which the presence and amount of miRNAs in the sample may be assayed by techniques in which the miRNAs in question are extracted from the sample. Such methods may make use of many of the assay techniques suitable for use in methods where the sample is a body fluid sample.

Alternatively, methods disclosed herein in which the sample is a tissue sample may employ assays in which the presence and amount of miRNAs is determined while the miRNAs remain *in situ.* A range of histological techniques known to the skilled person may be employed. Merely by way of example, the presence and location of miRNAs within a tissue sample may be determined by techniques using *in situ* hybridisation. Without wishing to be bound by any hypothesis, the inventors believe that at least some of the miRNAs referred to above may be involved in tumourigenesis, and so the ability to determine their location *in situ* may be advantageous monitoring the progression of brain cancer.

### "Assaying"

The methods of the disclosure involve assaying samples from a subject in order to determine the amount of selected miRNAs that are found in the sample. Generally, the assays used to detect the miRNAs may be of any sort known to those skilled in the art as suitable for the detection of nucleic acids (such assays allowing a simple assessment that "some amount" or "no amount" of the miRNA in question is present), and preferably may allow quantification of the selected miRNA(s).

The assaying methods of the disclosure may involve the formation of complexes between naturally occurring miRNA molecules in a patient sample and non-natural agents. The non-natural agents may incorporate moieties or other suitable means that allow their detection when complexed with the miRNAs from the patient sample. Assaying for the miRNA in the methods of the disclosure may involve detection of these complexes formed between miRNA and a non-naturally occurring agent, such as a synthetic oligonucleotide, labelled antibody, or the like.

The assays used in the methods of the disclosure may determine the amount of the miRNA present in the sample directly. Suitable assays may, for example, involve labelling only to the native miRNA present in the sample.

Alternatively, the assays used in the methods of the disclosure may indirectly determine the amount of the miRNA present in the sample. By this is meant that an assay may be used in which a proxy for the miRNA is produced, and the amount of this proxy produced determined, thereby indirectly allowing quantification of the miRNA.

By way of example, the assay used to determine the presence of the miRNA may comprise an amplification step in which miRNA in the patient sample is used as a template for the generation of artificial nucleic acid molecules, and the presence and quantity of the artificial nucleic acid molecules present assessed, thus allowing the amount of the at least one miRNA present in the sample to be determined.

Suitable examples of assays using such amplification steps will be known to those skilled in the art. The amplification step may utilise quantitative reverse transcriptase polymerase chain reaction (qRT-PCR). It will be appreciated that many such techniques, including the example of qRT-PCR referred to above, will bring about the production of complementary DNA (cDNA) molecules - artificial nucleic acid sequences that are not found in nature. Such artificial nucleic acid sequences may be isolated from naturally occurring sequences as part of the assay.

### "Brain cancer"

The inventors believe that the methods of the disclosure are applicable in respect of a wide variety of brain cancers. Merely by way of example, the brain cancer referred to in conjunction with the methods of the disclosure may be selected from the group consisting of: gliomas; meningiomas; pituitary adenomas; and nerve sheath tumours. As will be appreciated from consideration of the experimental results described herein, the methods of the invention are ones in which the brain cancer referred to is glioma.

The invention will now be further described with reference to the following experimental results and accompanying Figures, in which:
Figure 1 is a schematic representation of the spin column method used to extract miRNA used in generating the Experimental Results.
Figure 2 shows results achieved on comparison of miRNA expression in U87MG and SVGp12 cells. Panel A of Figure 2 shows MiRNA expression of U87MG cells, in which 26 miRNAs were down regulated compared to SVGp12 cells. Panel B of Figure 2 shows the 10 most down-regulated miRNAs (circles with heavier outline in blue). Panel C of Figure 2 shows fold changes in Hsa- miR-101-3p, hsa-miR-29b-3p, hsa-miR-328 and hsa-miR-9-5p, all of which were up-regulated compared to SVGp12 (non-cancerous cells serving to provide a reference value, for the purposes of the methods of the present invention).

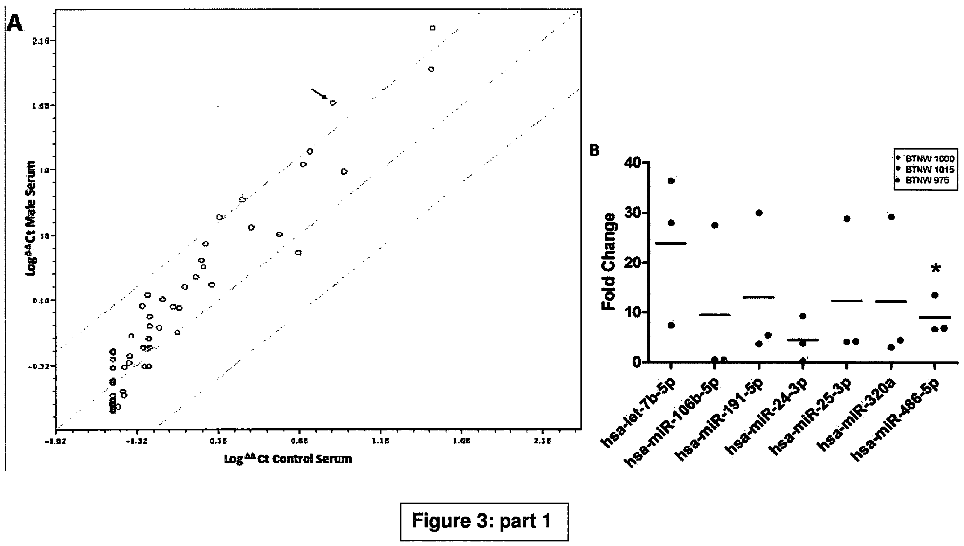

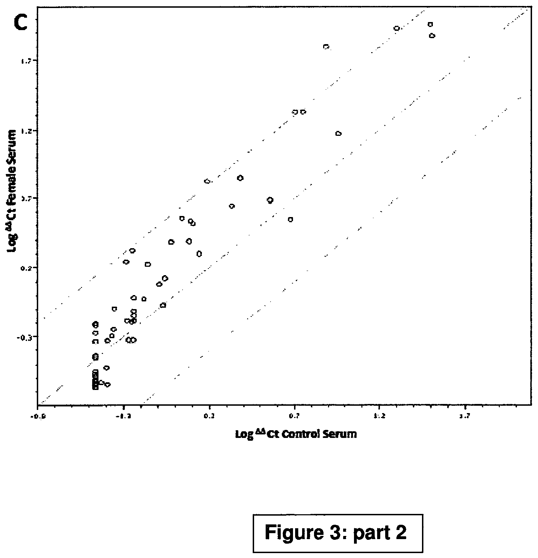

Figure 3 shows results achieved on comparison of miRNA expression in serum samples from male and female subjects. Panel A of Figure 3 illustrates that four microRNAs showed a 4-fold increase in expression in male serum when compared to control serum (red circles above the upper diagonal line). Two miRNAs exhibiting the highest levels of expression were common between male and female, miR-486-5p and 25-3p. Panel B of Figure 3 shows that seven miRNAs were up-regulated in the male serum compared to the controls. The female serum exhibited an increase in 11 miRNAs. Panel C compares miRNA expression in serum samples (cancer and control) from female subjects. MiR-451, 486-5p, 92a-3p and 25-3p exhibited the highest expression in female serum.

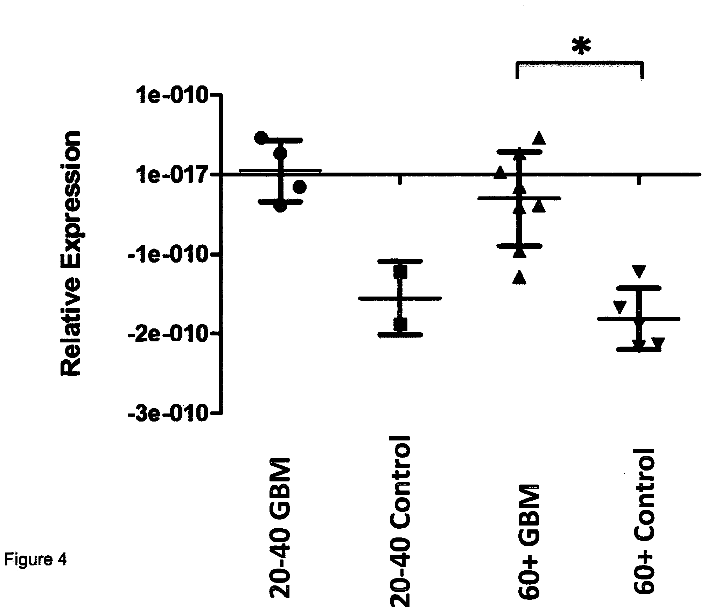

Figure 4 show results achieved on comparison of Hsa-miR-34a-5p expression in serum samples from subjects grouped by age (aged 20 to 40 years, or aged 60 years or more). Expression of miR-34a is up-regulated in patients with the brain cancer glioblastoma, as compared to controls. This elevation is statistically significant when patients aged 60 years or over are compared to age-matched controls.

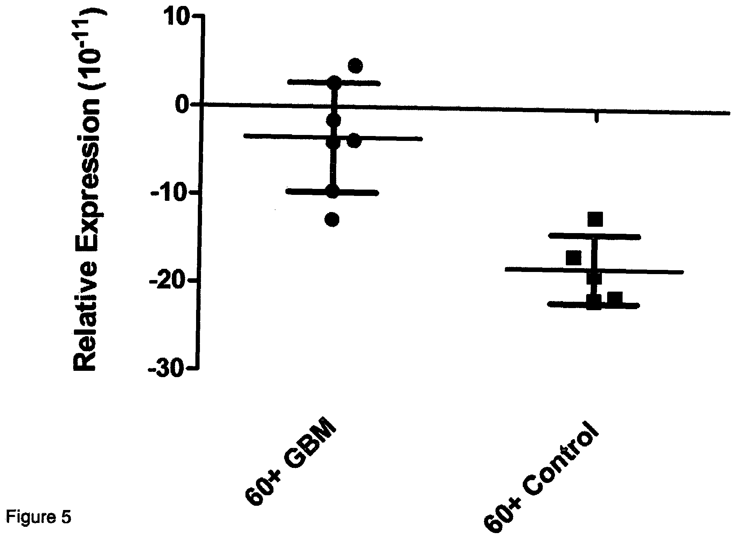

Figure 5 replicates the results illustrated in Figure 4 in respect of the comparison of Hsa-miR-34a-5p expression in cancer patients and control subjects aged 60 years or more.

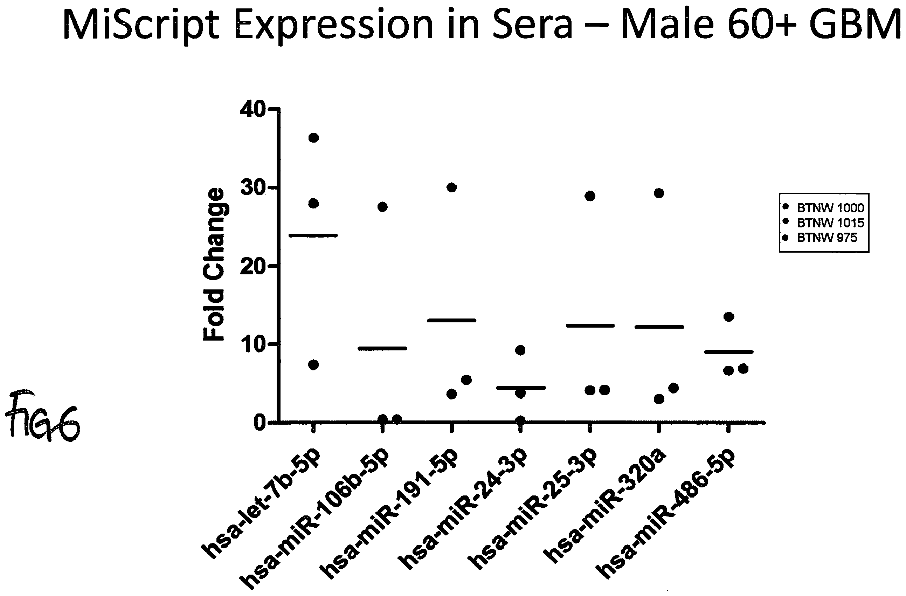

Figure 6 illustrates miRNA with the greatest fold change in the serum of individual male 60+ GBM samples. Seven miRNA exhibited a greater than 2-fold increase in male 60+ GBM serum samples. Let-7b-5p showed the greatest increased fold change in two of the three samples, BTNW1000 showed the highest increased fold change for all seven miRNA.

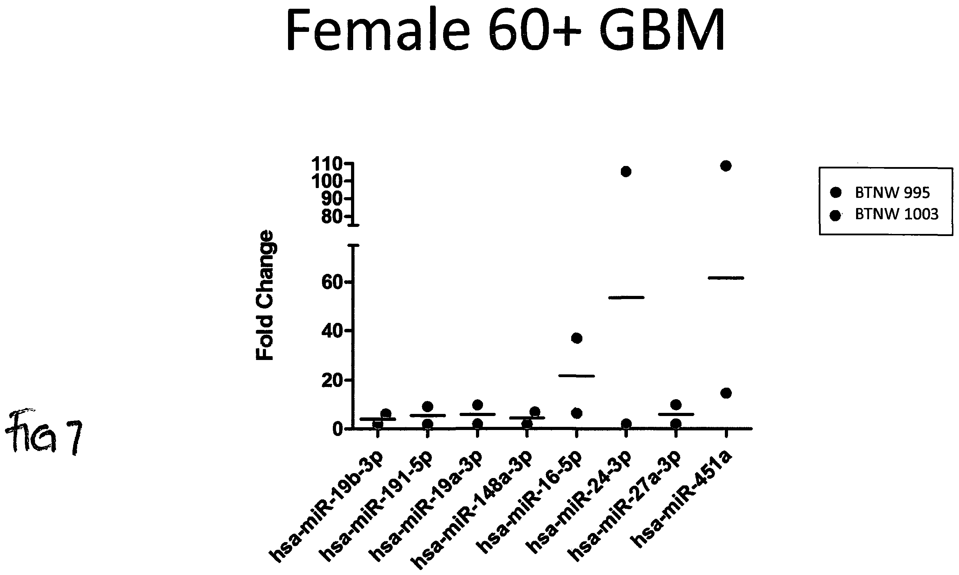

Figure 7 illustrates miRNA with the greatest fold change in the serum of individual female 60+ GBM samples. Eight miRNA showed a greater than 2 fold change in at least one of the female GBM samples compared to the control. MiR-24-3p and miR-451a showed the greatest increased fold change with miR-451a exhibiting the highest increased fold change for both samples.

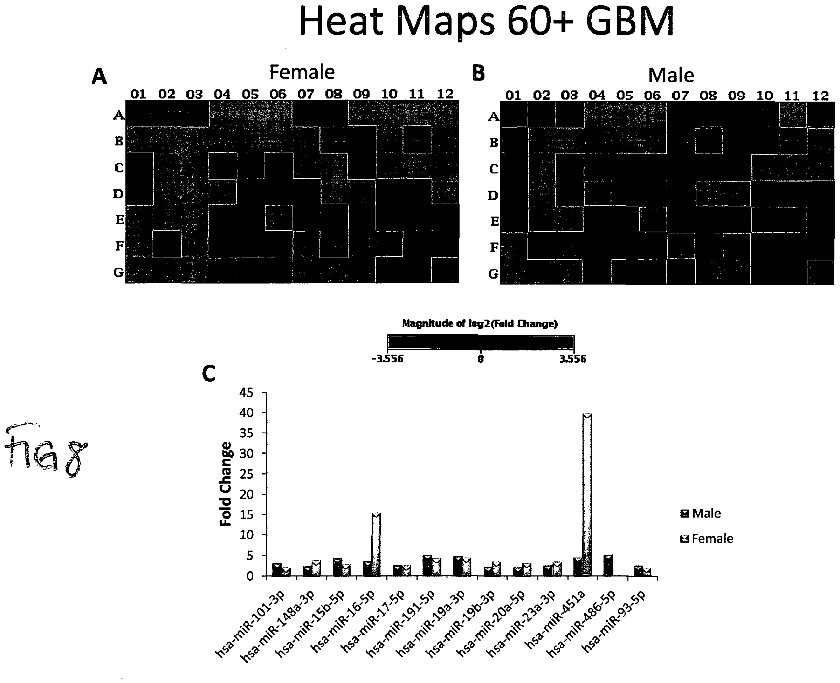

Figure 8 illustrates combined expression of miRNA isolated from the serum of GBM patients over the age of 60.

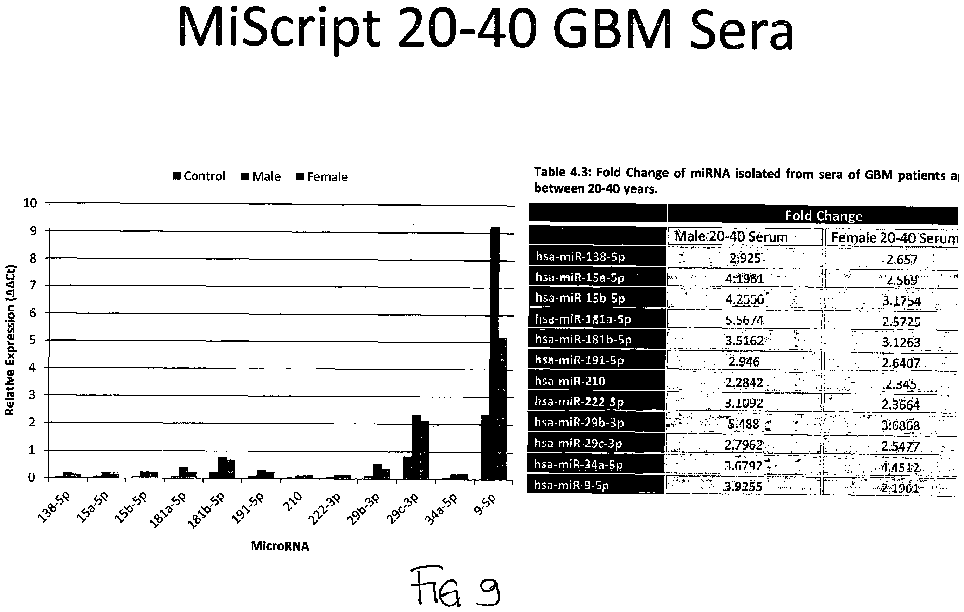

Figure 9 illustrates expression of miRNA in sera of GBM patients aged between 20 - 40 years. qPCR analysis using the miScript brain cancer panel identified 12 miRNAs with altered expression in the sera of GBM patients aged between 20-40 years. MiR-9-5p had the highest expression of all 12 miRNAs and 29c-3p had the second highest expression of the group.

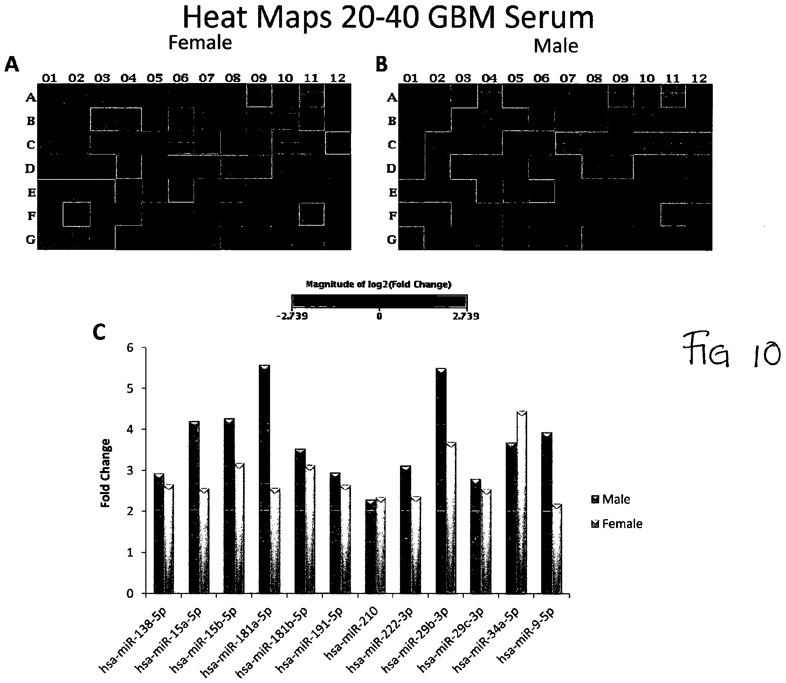

Figure 10 illustrates expression of miRNA isolated from the sera of GBM patients aged between 20-40 years.

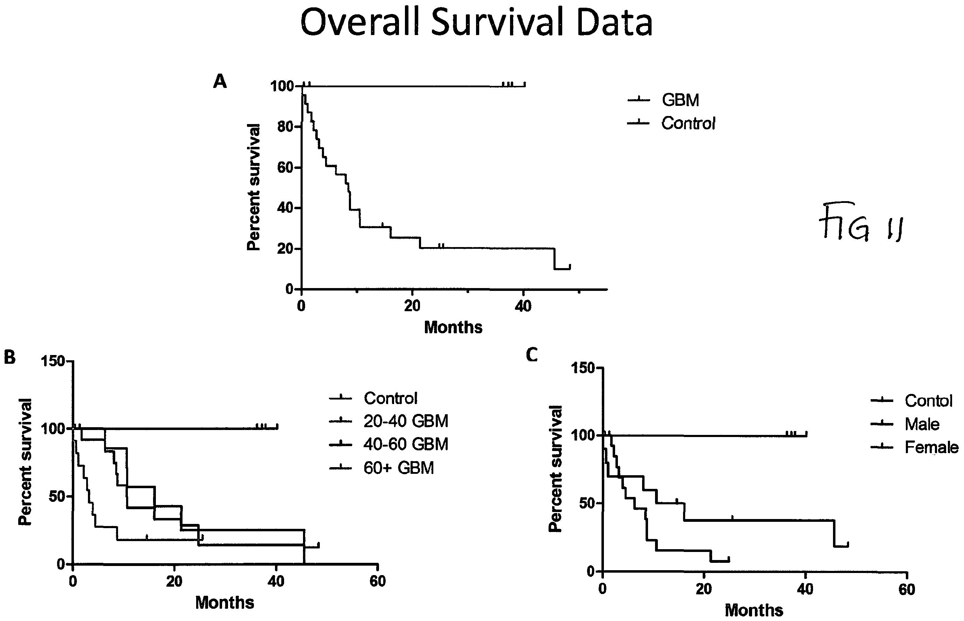

Figure 11 illustrates Kaplain-Meier Graphs of overall survival of GBM patients used in this study. A) Overall survival of GBM patients compared to control patients without GBM. Median survival for GBM patients was 8.51 months. Survival was determined using Chi Square p = 0.0015. B) Overall survival of GBM patients by age group. Median survival was 10.57 months for patients aged 20-40, 16.09 months for patients aged 40-60 and 3.12 months for patients aged over 60 years p = <0.0001 determined by Log rank, Mantel-Cox test. C) Overall survival of GBM patients by gender, median survival was 6.3 months for male patients and 13.31 months for female patients, p = 0.0022. Significance was determined by Log rank, Mantel Cox text.
Figure 12 shows relative expression of miR-34a in sera obtained from patients aged over 60 years. GBM patients over the age of 60 showed an up-regulation of serum miR-34a compared to aged matched controls. Relative expression is shown as 2^-ΔCt. p=<0.05
Figure 13 shows Kaplan-Meier Graph of GBM patients over the age of 60 with high and low miR-34a expression. GBM patients with a greater than 2 fold change in expression had a median survival of 10.6 months and patients with a less than 2 fold change in miR-34a expression had a median survival of 8.78 months. Median survival was significantly different between the groups p = 0.0051, determined by Log rank, Mantel-Cox test
Figure 14 shows scatter graph of tissue miR-34a expression by age. Analysis of the TCGA dataset identified a correlation between miR-34a expression and age of patients.
Figure 15 shows survival curve of patients with high and low expression miR-34a in tissue. Analysis of the TCGA dataset showed higher expression of miR-34a was associated with a poorer prognosis.

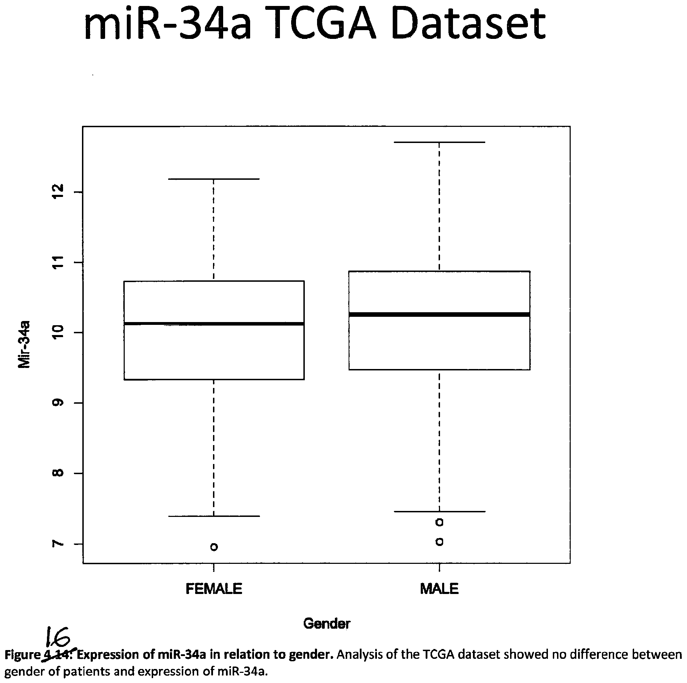

Figure 16 shows expression of miR-34a in relation to gender. Analysis of the TCGA dataset showed no difference between gender of patients and expression of miR-34a.
Figure 17 illustrates relative expression of miR-20a in sera of GBM patients compared to controls. A subpopulation of GBM patients showed an up-regulation in serum miR-20a expression compared to age and sex matched controls. Certain patients showed no up-regulation in serum miR-20a expression. Relative expression is shown as 2^-ΔCt. p=<0.05.
Figure 18 illustrates overall survival of GBM patients with <2 fold change and >2 fold change in miR-20a expression compared to controls. GBM patients with less than a 2 fold change in miR-20a expression had a median survival of 4.45 months whereas patients with a greater than two fold change had a median survival of 21.39 months. Overall survival was significantly different p = 0.0001 as determined by Log rank, Mantel-Cox test.

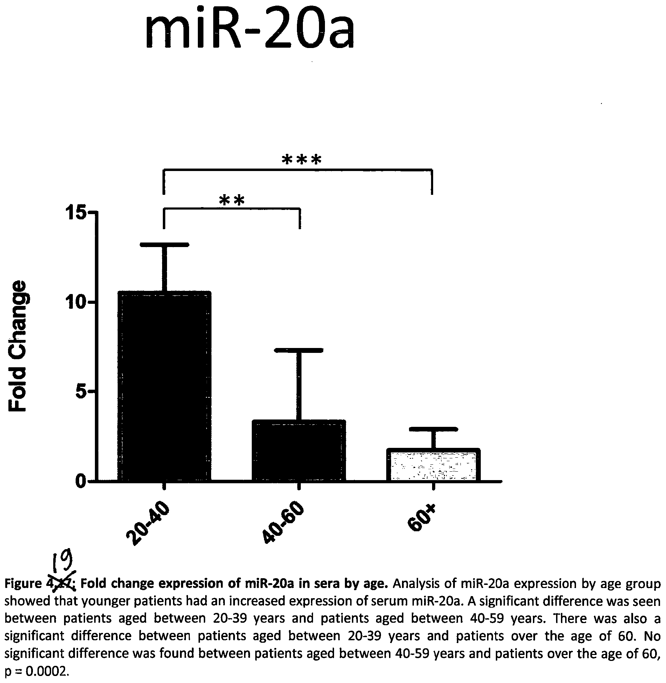

Figure 19 shows fold change expression of miR-20a in sera by age. Analysis of miR-20a expression by age group showed that younger patients had an increased expression of serum miR-20a. A significant difference was seen between patients aged between 20-39 years and patients aged between 40-59 years. There was also a significant difference between patients aged between 20-39 years and patients over the age of 60. No significant difference was found between patients aged between 40-59 years and patients over the age of 60, p = 0.0002.

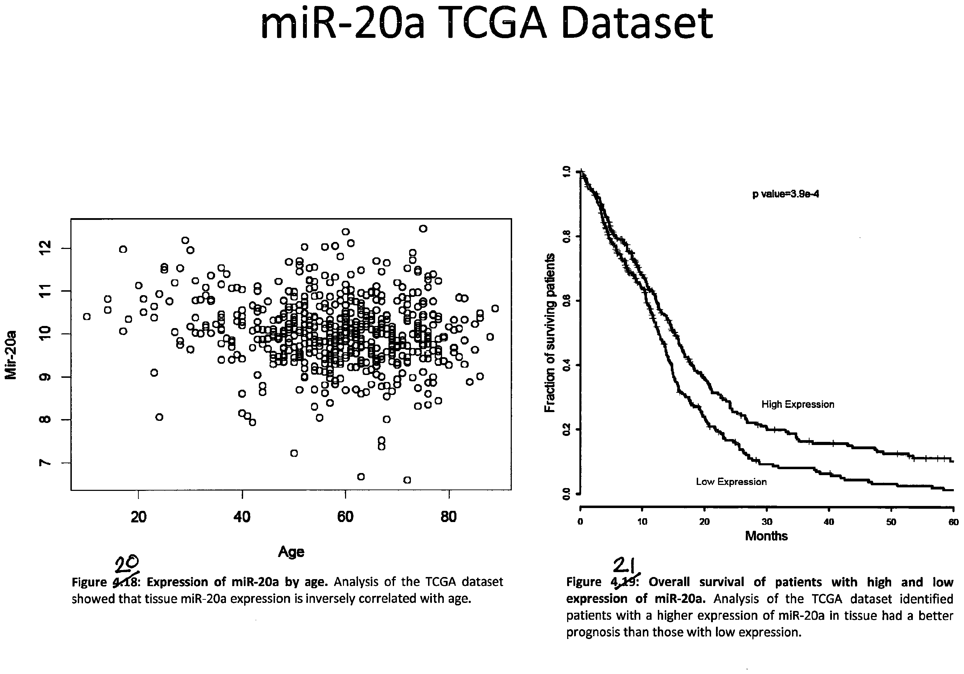

Figure 20 shows expression of miR-20a by age. Analysis of the TCGA dataset showed that tissue miR-20a expression is inversely correlated with age.
Figure 21 illustrates overall survival of patients with high and low expression of miR-20a. Analysis of the TCGA dataset identified patients with a higher expression of miR-20a in tissue had a better prognosis than those with low expression.

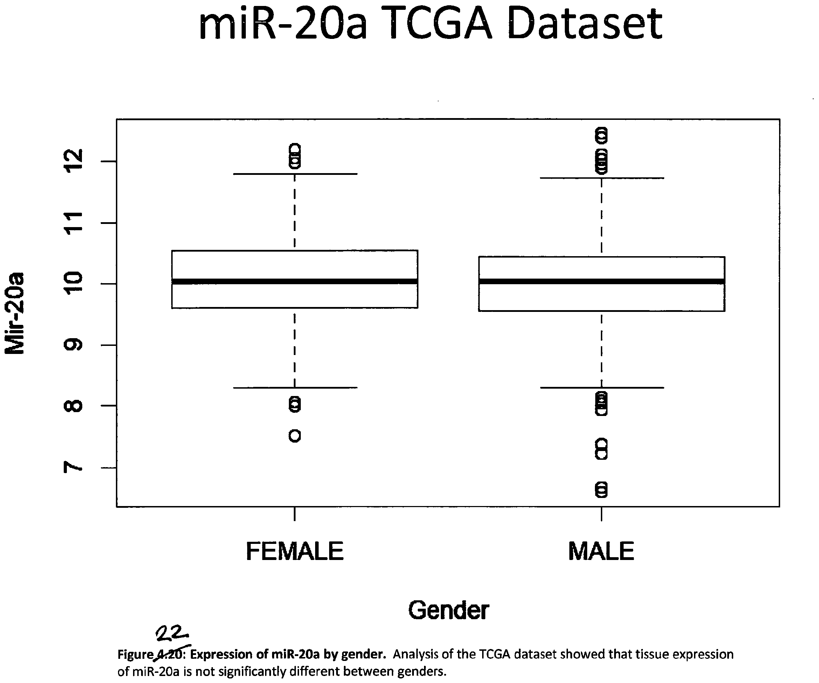

Figure 22 expression of miR-20a by gender. Analysis of the TCGA dataset showed that tissue expression of miR-20a is not significantly different between genders.
Figure 23 illustrates Relative expression of miR-92a in the serum of male GBM patients compared to sex-matched controls. Male GBM patients showed a down-regulation in serum miR-92a expression compared to sex-matched controls. Relative expression is shown as 2^-ΔCt. p=<0.05
Figure 24 illustrates Kaplan-Meier graph of male GBM patients with high and low expression of miR-92a compared to sex-matched controls. Median survival for male GBM patients with high miR-92a expression was 8.78 months, patients with low miR-92a expression was 5.375 months. Overall survival was significantly different between the groups, p = 0.0025 as determined by Log rank, Mantel-Cox test.
Figure 25 shows expression of miR-92a by age. Analysis of the TCGA dataset showed that expression of tissue miR-92a is inversely correlated with age.
Figure 26 shows survival curve of GBM patients with high and low expression of miR-92a. Analysis of the TCGA dataset showed patients with a higher expression of tissue miR-92a had a better prognosis than those with a lower expression

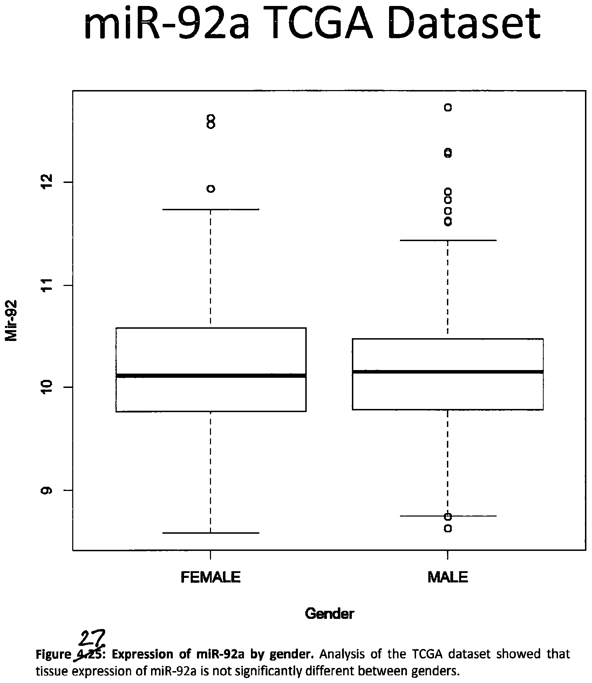

Figure 27 illustrates expression of miR-92a by gender. Analysis of the TCGA dataset showed that tissue expression of miR-92a is not significantly different between genders

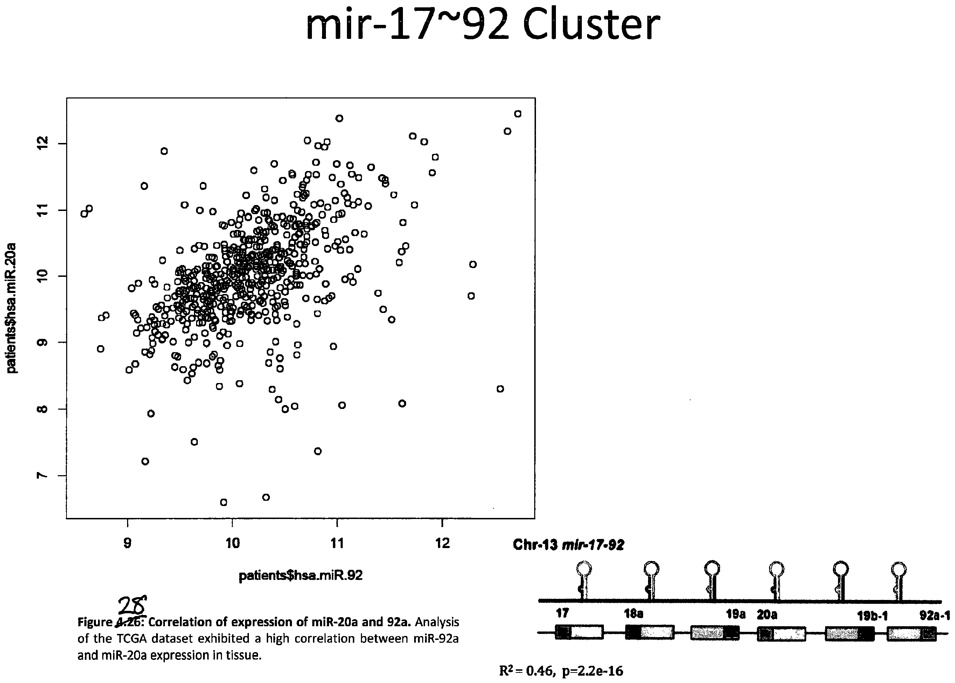

Figure 28 shows correlation of expression of miR-20a and 92a. Analysis of the TCGA dataset exhibited a high correlation between miR-92a and miR-20a expression in tissue.

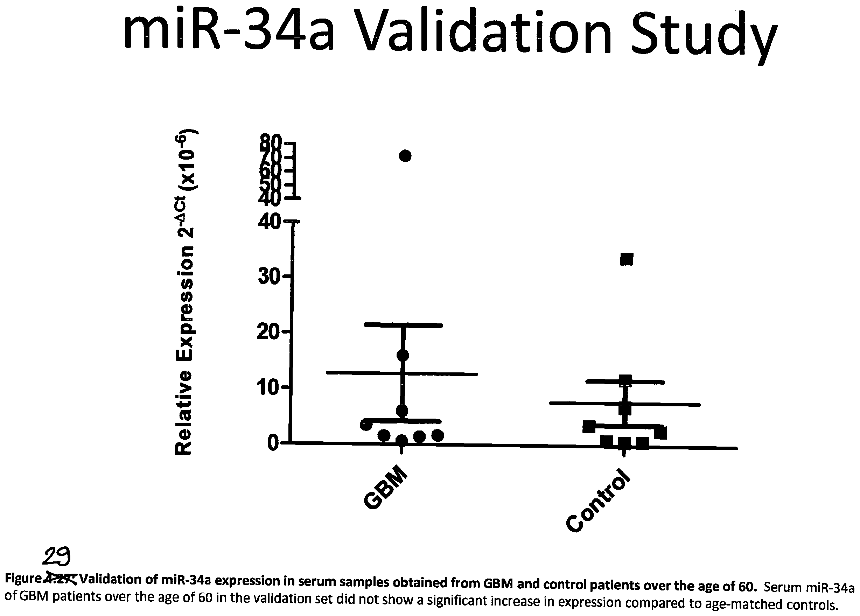

Figure 29 illustrates validation of miR-34a expression in serum samples obtained from GBM and control patients over the age of 60. Serum miR-34a of GBM patients over the age of 60 in the validation set did not show a significant increase in expression compared to age-matched controls
Figure 30 illustrates validation of miR-20a expression in serum samples obtained from GBM and control patients. Expression of miR-20a in the validation set again showed two populations of GBM patients, with and without a 2 fold increase in serum miR-20a expression. There was a significant up-regulation of miR-20a between the two groups of GBM patients as well as between patients with a greater than 2 fold increase in expression and the control group, p= <0.05.
Figures 31 to 40 illustrate results obtained from investigation of miRNA expression in glioma cancer cells using samples from The Cancer Genome Atlas (TCGA).

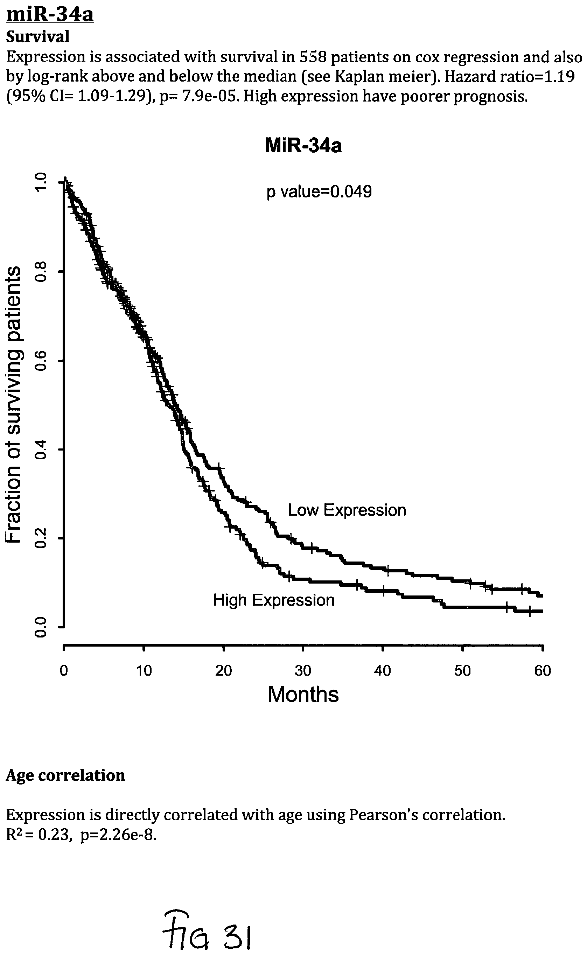

Figure 31 illustrates miR-34a survival rates Expression is associated with survival in 558 patients on cox regression and also by log-rank above and below the median (see Kaplan meier). Hazard ratio=1.19 (95% Cl= 1.09-1.29), p= 7.9e-05. High expression have poorer prognosis.

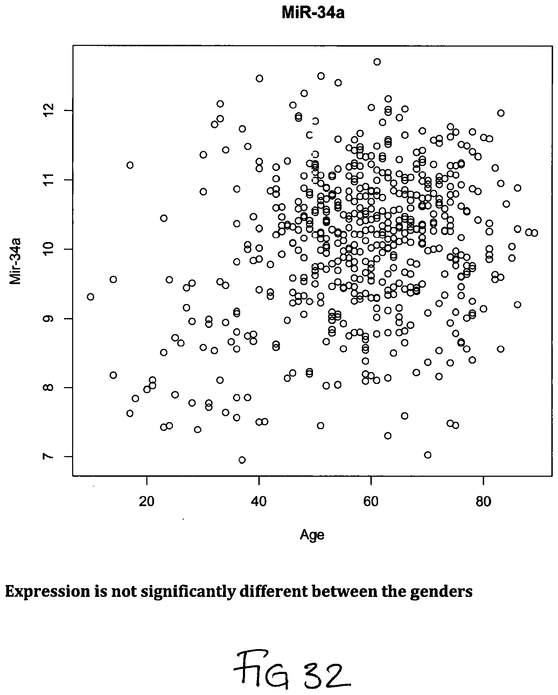

Figure 32 show age correlation for miR-34a: Expression is directly correlated with age using Pearson's correlation.
   R2= 0.23, p=2.26e-8.

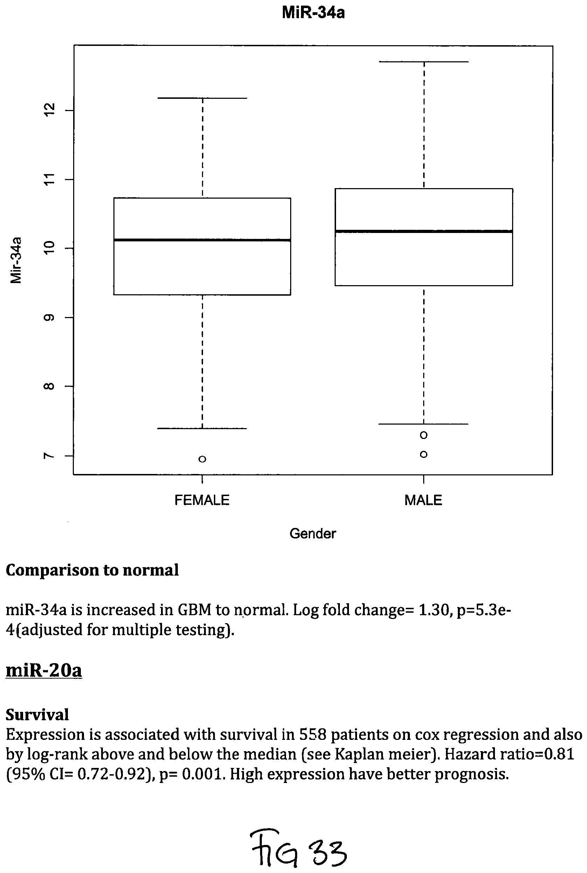

Figure 33 shows that expression of miR-34a is not significantly different between the genders. Comparison of miR-20a from cancer cells to normal. miR-34a is increased in GBM. Log fold change=1.30, p=5.3e-4 (adjusted for multiple testing).

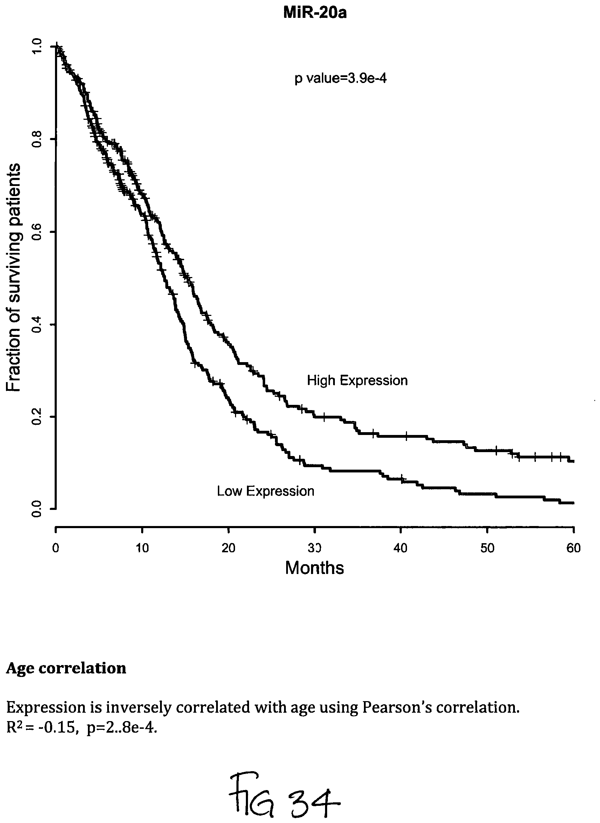

Figure 34 shows miR-20a data regarding survival. Expression is associated with survival in 558 patients on cox regression and also by log-rank above and below the median (see Kaplan Maier). Hazard ratio=0.81 (95% CI=0.72-0.92), p=0.001. Patients with high expression have better prognosis.

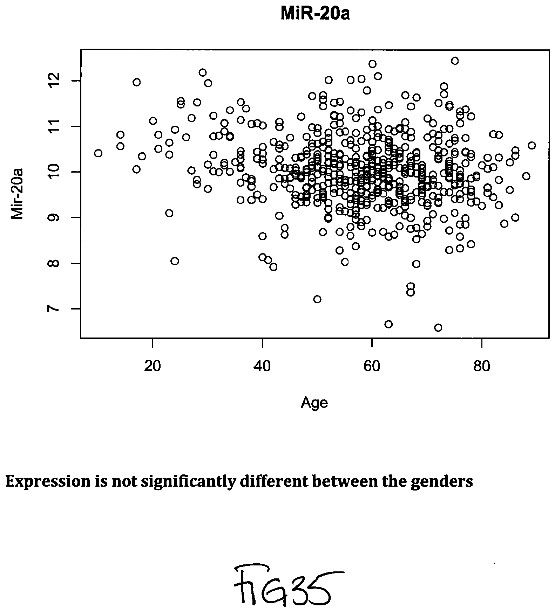

Figure 35 show age correlation in respect of expression of miR-20a. Expression is inversely correlated with age using Pearson's correlation. R2=-0.15, p=2..8e-4

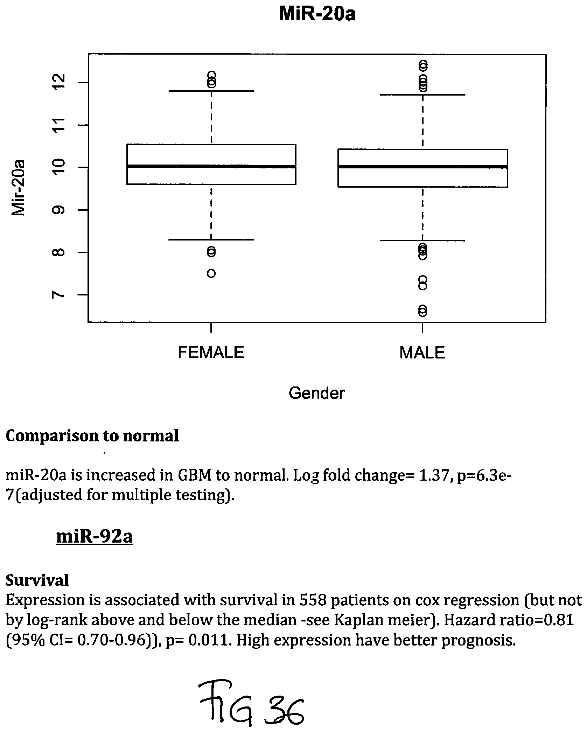

Figure 36 shows that expression of miR-20a is not significantly different between genders. Comparison to normal reveals that miR-20a is increased in GBM to normal. Log fold change 1.37, p=6.3e-7 (adjusted for multiple testing).

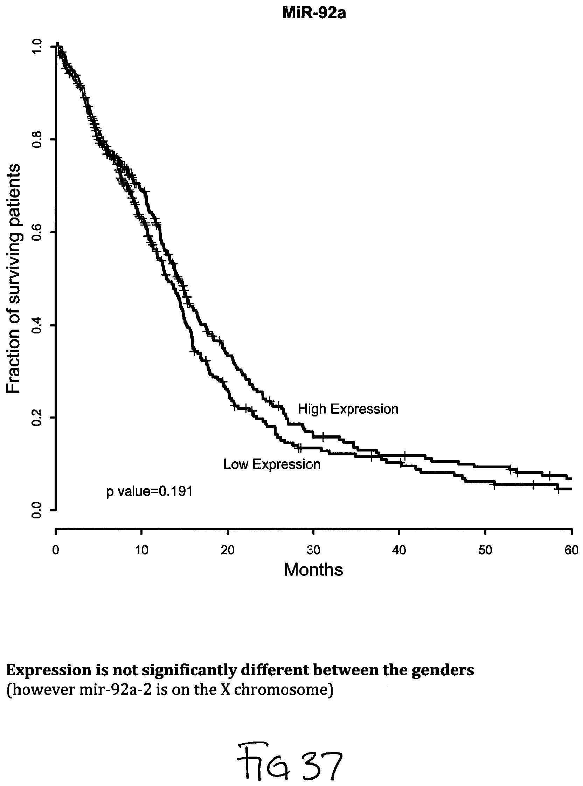

Figure 37 shows data regarding miR-92a expression and survival. Expression is associated with survival in 558 patients on Cox regression (but not by log-rank above and below the median - se Kaplan Maier). Hazard ratio=0.81 (95%CI=0.70-0.96), p=0.011. High expression is associated with better prognosis.

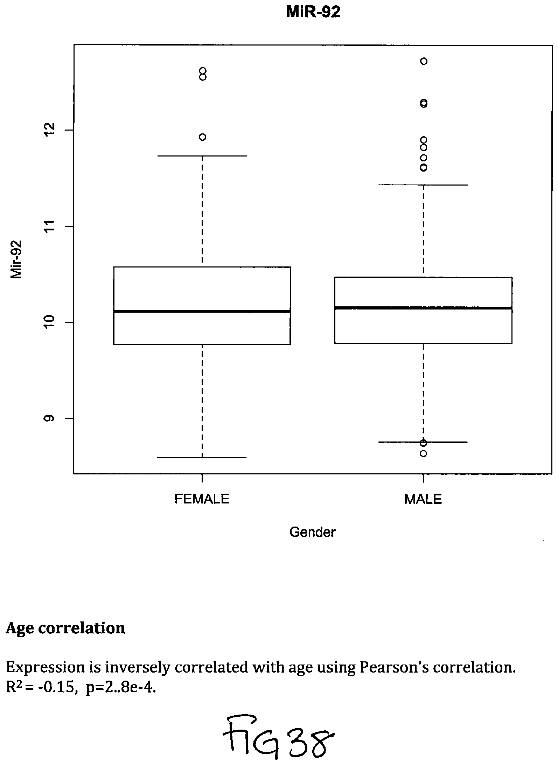

Figure 38 illustrates that expression of miR-92a is not significantly different between the genders.

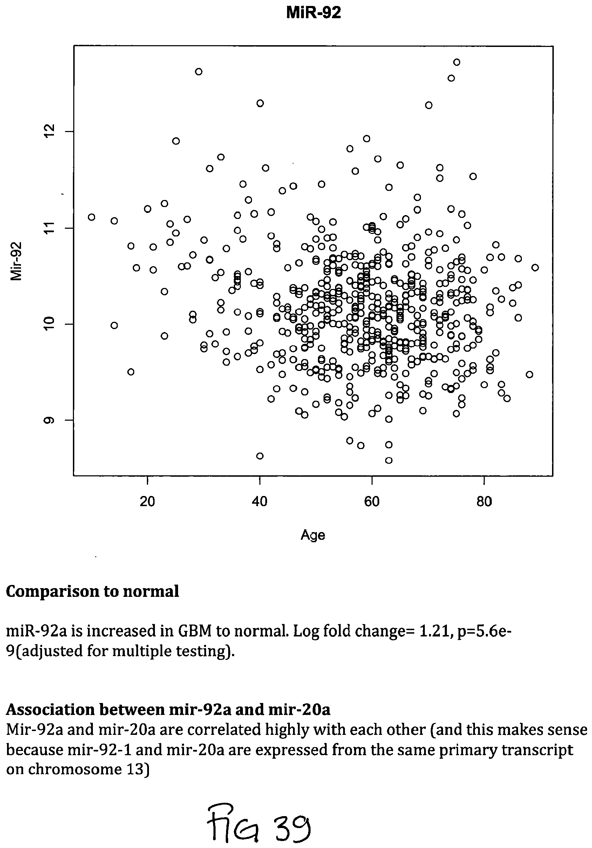

Figure 39 illustrates that expression of miR-92a is inversely correlated with age using Pearson's correlation. R2=-0.15; p=2..8e-4.
   Comparison to normal reveals that miR-92a is increased in GBM as compared to normal. Log fold change = 1.21, p=5.6e-9 (adjusted for multiple testing).

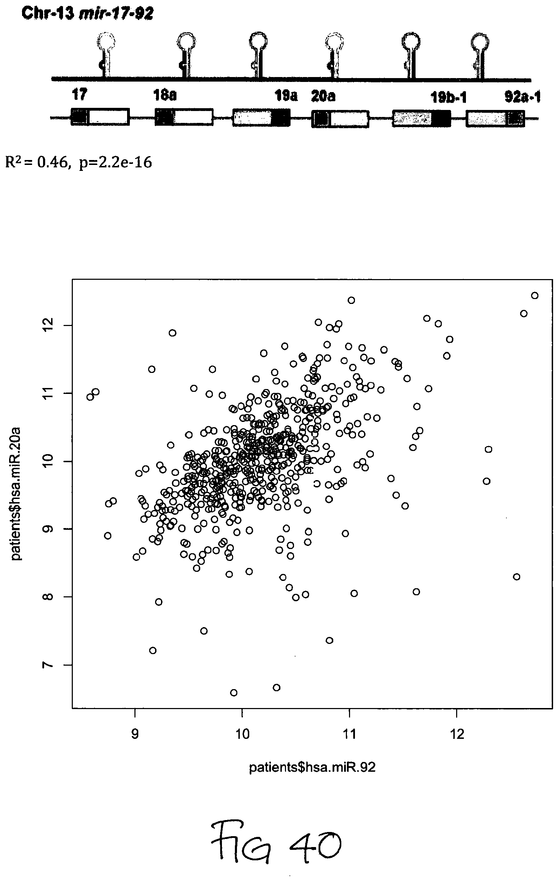

Figure 40 illustrates the association between miR-92a and miR-20a. These micro RNA markers are correlated highly with one another (and this makes sense because mir-92 and mir-20a are expressed from the same primary transcript on chromosome 13).

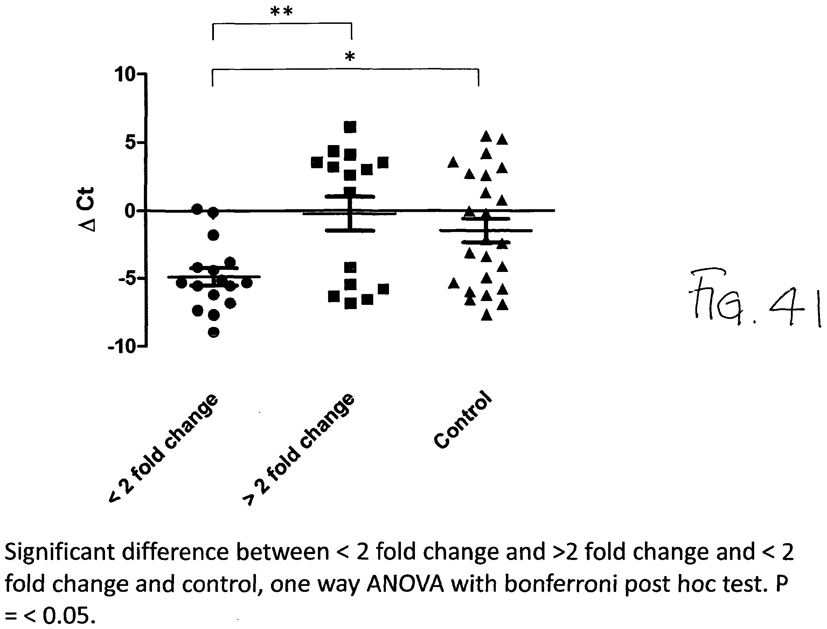

Figure 41 illustrates analysis of results obtained from data regarding miR-20a levels in serum samples, indicating significant difference between cancer patients with a < 2 fold change and those with a >2 fold change and cancer patients with < 2 fold change and control, one way ANOVA with bonferroni post hoc test. P = < 0.05.

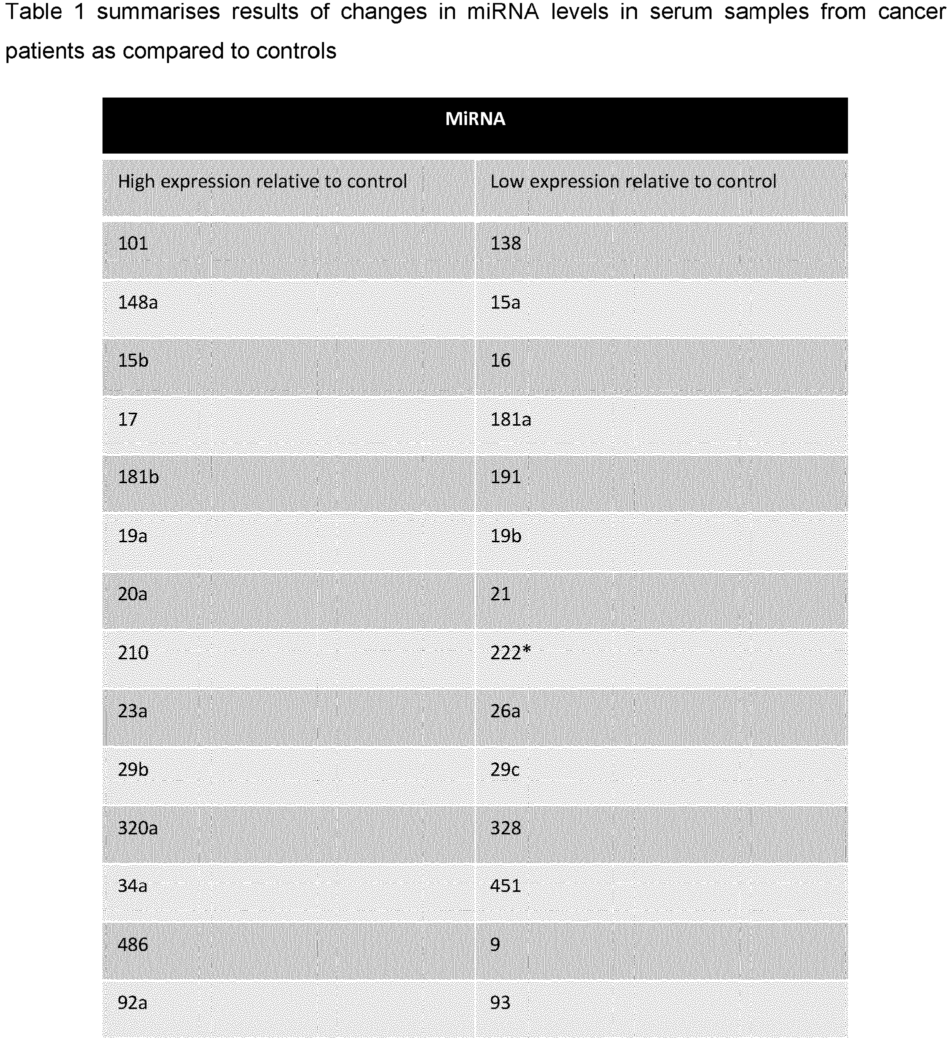

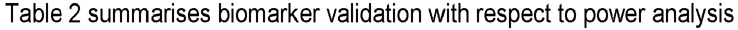

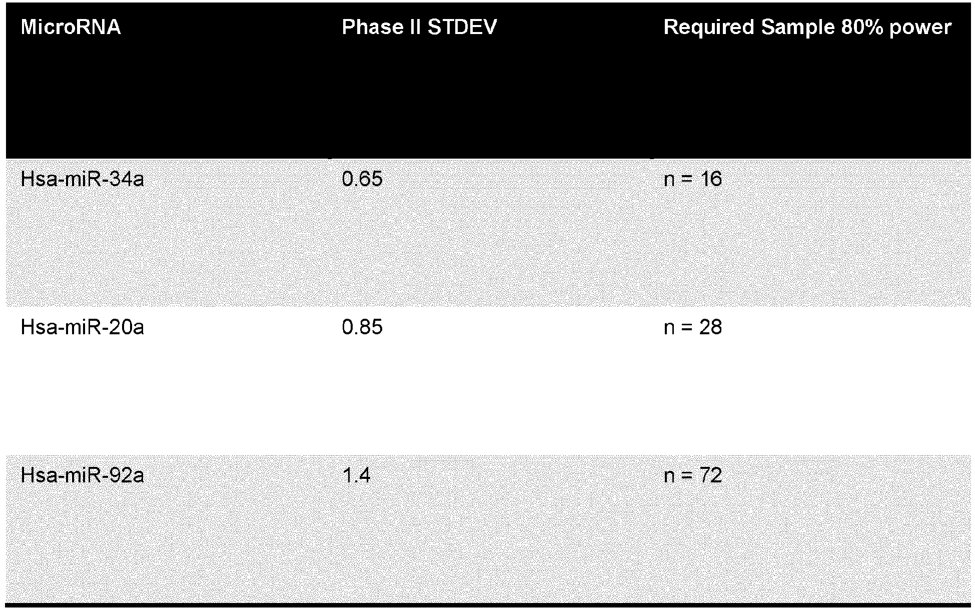

### Experimental Results

### Study 1

### Introduction

MicroRNAs (miRNA) are small non-coding RNAs which play a role in post-transcriptional regulation of gene and protein expression. MiRNAs exhibit disease specific expression, which can be used to provide information about a particular biological state, such as glioma. Changes in miRNA expression in gliomas can be measured following the isolation of glioma specific exosomes released into the circulation.

**Aim:** To identify a panel of miRNAs isolated from the circulation for diagnosis, prognosis and prediction of response to treatment of glioma.

### Samples

**Patient samples:** Serum was obtained post-operatively from male and female patients aged over 60 years, with a diagnosis of glioblastoma. Non-cancerous serum was obtained from age and sex matched patients undergoing elective surgery, postoperatively. Information on the type of medication being used by these patients was also obtained to account for any effects these drugs may have on miRNA expression.

Levels of miRNAs in tissue samples from patients with cancer, such a glioblastoma, were assessed with reference to the cancer genome atlas (TCGA) is an online bioinformatics repository containing data such as miRNA expression in patient tissues.

**Cell lines:** U87MG, grade IV glioma and SVGp12, non-cancerous astrocyte, cell lines (ECACC) were cultured as a monolayer in standard conditions until 80% confluent. Cells were then harvested and miRNA isolated.

### Methods

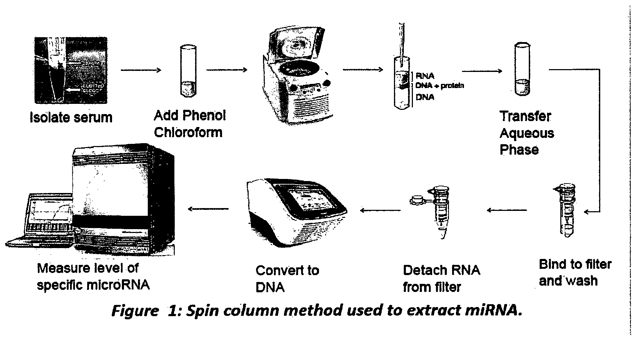

MiRNA was extracted using a spin column method (Figure 1). Following extraction, the expression of 82 miRNAs associated with brain neoplasms were determined using qRT-PCR. Statistical analysis was performed on the male serum group using a student's t-test and a p-value of 0.05 or less was considered significant.

### MicroRNA Extraction of Serum Samples

100µl per sample of RNase DEPC treated water was heated in a heat block to 95°C

### Trizol LS Protocol

750µl of Trizol LS (invitrogen) was added to 250µl serum and homogenised by pipetting up and down.

The sample was centrifuged at 4°C, 12,000 xg for 10 minutes to remove high molecular weight DNA and protein.

The sample was transferred to a new tube and 3.5µl of cel-miR-39 spike in was added and the sample incubated for five minutes at room temperature.

200µl of chloroform was added and shook vigorously by hand for 15 seconds and incubated at room temperature for 10 minutes.

The sample was then centrifuged at 4°C, 12,000 xg for 15 minutes to separate the phases, the aqueous phase was removed and transferred to a fresh tube, noting the volume removed.

### Continue with miRVana Protocol

1.25 volumes of ethanol were added to the samples and pipetted onto mirVana spin columns, 700µl at a time and spun at 10,000 xg for 15 seconds.

500µl of wash solution 1 was added to the filter and centrifuged at 10,000 xg for 10 seconds. 500µl of wash solution 2/3 was added to the filter and centrifuged at 10,000 xg for 10 seconds. 500µl of wash solution 2/3 was added and centrifuged at 10,000 xg for 10 seconds.

The spin column was then centrifuged for one minute at 10,000 xg to remove residual fluid from the filter. The filter was transferred to a new tube and 100µl of the RNase free DEPC treated water was added to the filter and spun for 30 seconds at 10,000 xg.

### Reverse Transcription

Reverse transcription was performed using the qiagen miscript II RT kit. 12.5 ng/µl of total RNA was used in the reverse transcription reaction.

The kit components were thawed on ice and prepared as below and gently mixed.

| **Component** | **Volume** |
|---|---|
| 5x miscript HiSpec buffer | 4µl |
| 10x miscript nucleics mix | 2µl |
| Rnase free water | Variable |
| Miscript reverse transcriptase mix | 2µl |
| Template miRNA | Variable |
| **Total Reaction Volume** | **20µl** |

The reaction was incubated at 37°C for 60 minutes followed by 5 minutes at 95°C. Following reverse transcription, 20µl of the cDNA was diluted in 200µl of RNase free, DEPC treated water.

### qPCR Reaction

Miscript human brain cancer miRNA PCR arrays (MIHZ-108Z) were used for the qPCR reaction. The qPCR master mix was prepared using the miScript SYBR green PCR kit.

The kit components were thawed and prepared as below.

| | |
|---|---|
| 2x QuantiTect SYBR Green PCR master mix | 1375 µl |
| 10X miScript Universal Primer | 275µl |
| RNase free water | 1000µl |
| Template cDNA | 100µl |
| **Total Volume** | **2750µl** |

25µl of master mix was pipetted into the miscript miRNA array. The plate was centrifuged for 1 minute at 1000 xg. The qPCR reaction was performed using the parameters below for 40 cycles using the ABI 7500 qPCR machine. Dissociation analysis was performed following the qPCR reaction.

| Time | Temperature |
|---|---|
| 15 min | 95°C |
| 15 sec | 94°C |
| 30 sec | 55°C |
| 30 sec' | 70°C |
| | |

Data analysis was performed using the SABiosciences miScript miRNA PCR Data Analysis web portal. Data was normalised to cel-miR-39 spike in.

### Results

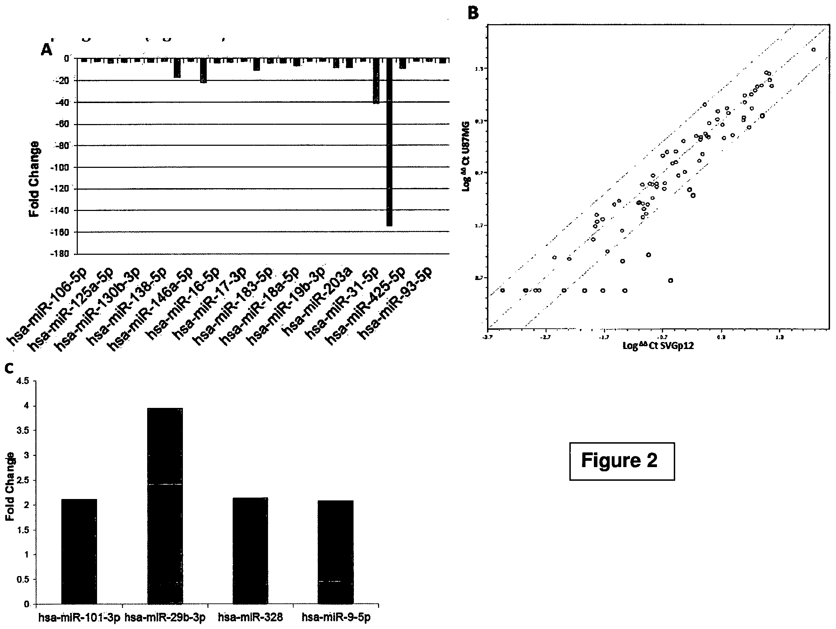

**Cell Lines:** Profiling of the U87MG cell line identified 26 miRNAs whose expression were down-regulated compared to SVGp12 (Figure 2A), and 4 miRNAs that were up-regulated (Figure 2C).

**Serum:** Seven miRNAs exhibited a 3-fold increase in expression in male serum compared to the control (Figure 3B) and four exhibited more than a 4-fold change (Figure 3A). Out of the seven, miR-486-5p was significantly up-regulated in the male cancerous group (p=0.01) (Figure 3A (arrow) and B). A comparison of the top four up-regulated miRNAs in male and female serum showed miR-486-5p and 25-3p up-regulated in both groups (Figure 3A and C).

### Discussion

MiRNA expression of both cell lines showed no similarity to the circulatory miRNAs isolated from serum. Inherent differences between immortalised cells and glioma expression *in vivo* could account for the contrast in miRNA expression. A study which profiled cancerous and non-cancerous tissue found a general down-regulation of miRNA expression in tumours, similar to that seen in the U87MG profile obtained in this study3.

### Conclusion and Future Work

A panel of circulatory miRNAs with altered expression in the serum of glioma patients was identified and will be tested on a larger sample set. In conclusion, the use of circulatory miRNA biomarkers could vastly improve the diagnosis of gliomas as well as provide invaluable prognostic information. In addition to improving clinical aspects of gliomas, these results will contribute to our understanding of the role of miRNAs in the pathology of glioma.

### Study 2

The aim of this second study was to identify circulating microRNA for use as biomarkers for glioma.

MicroRNA was isolated from serum of glioblastoma (n=26) and control patients (n=23), using phenol-chloroform extraction. Relative expression of microRNA was determined using qRT-PCR. Data were normalised using a synthetic spike in and analysed using the 2^{-ΔΔCt} method. Statistical significance was determined using a Mann-Whitney t-test and a p-value of <0.05 was considered significant.

Three microRNAs were identified as being differentially expressed in the serum of glioblastoma patients when compared to control serum. In confirmation of the results reported above, microRNA-20a was up-regulated in the serum of glioblastoma patients as compared to controls. Furthermore, individuals with a two fold increase in microRNA-20a had a better survival time than those with only a one fold increase shown by Kaplan-Meier survival analysis. Levels of miRNAs in tissue samples from patients with cancer, such a glioblastoma, were assessed with reference to the cancer genome atlas (TCGA), and this analysis indicated that, in 558 patient tissues, high levels of expression of micro-RNA-20a also correlated with better prognosis.

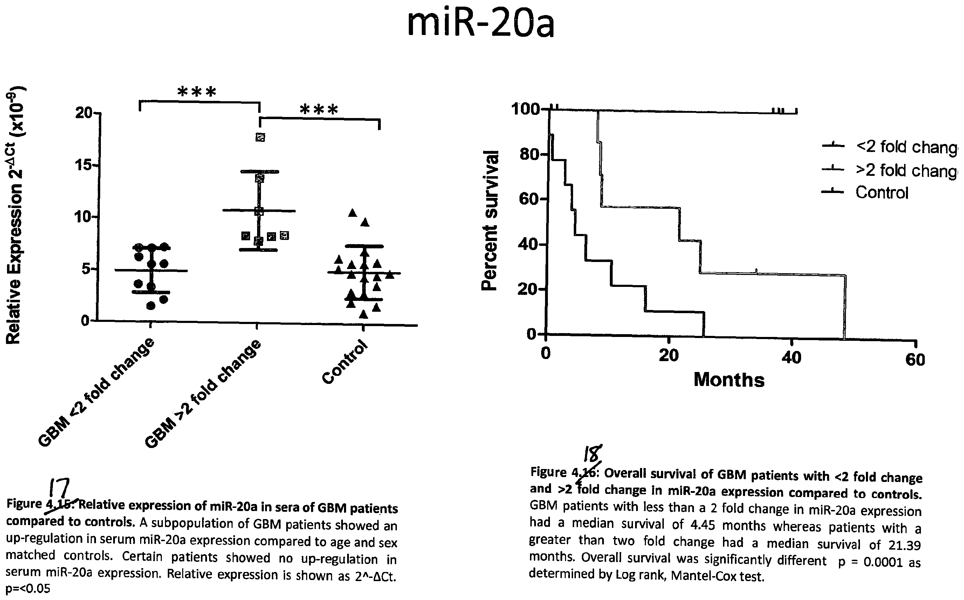

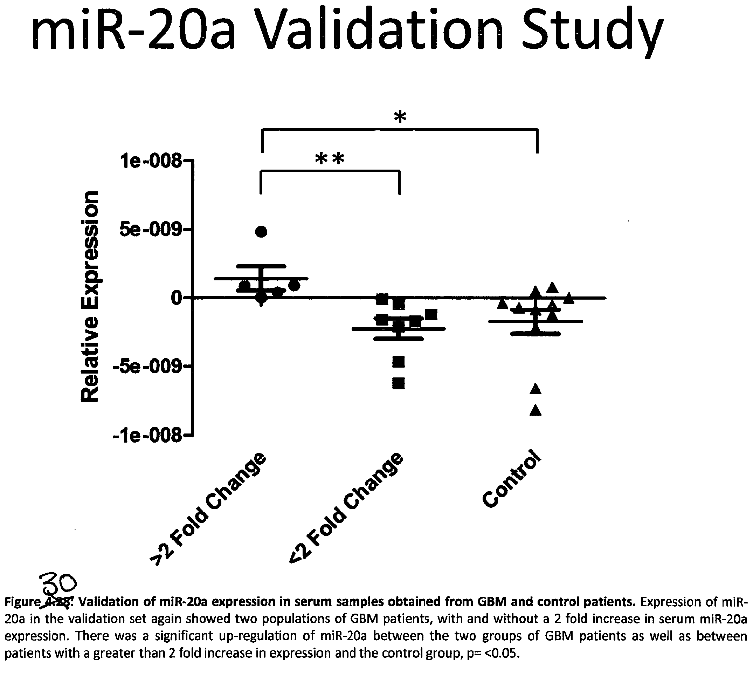

miRNA20a is a particularly promising prognostic biomarker. The level of miRNA20a in cancer patients is inversely correlated with age, but analysis using age and sex matched patients with glioblastoma multiforme (GBM) and controls has shown that a >2 fold increase in expression in GBM patients is correlated with a better prognosis. These results are illustrated in Figure 17 and 18. Power analysis shows that this is indicative of a clinically significant outcome (at 80% power), and this has been confirmed in an expanded patient cohort including another 28 patients (14 GBM and 14 control) Figure 30 and 41

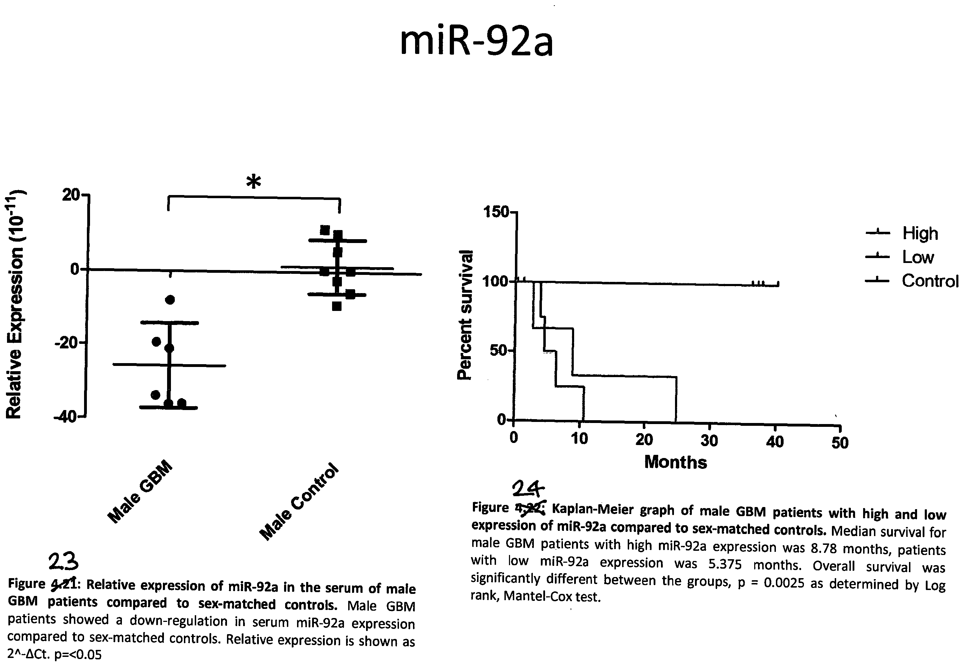

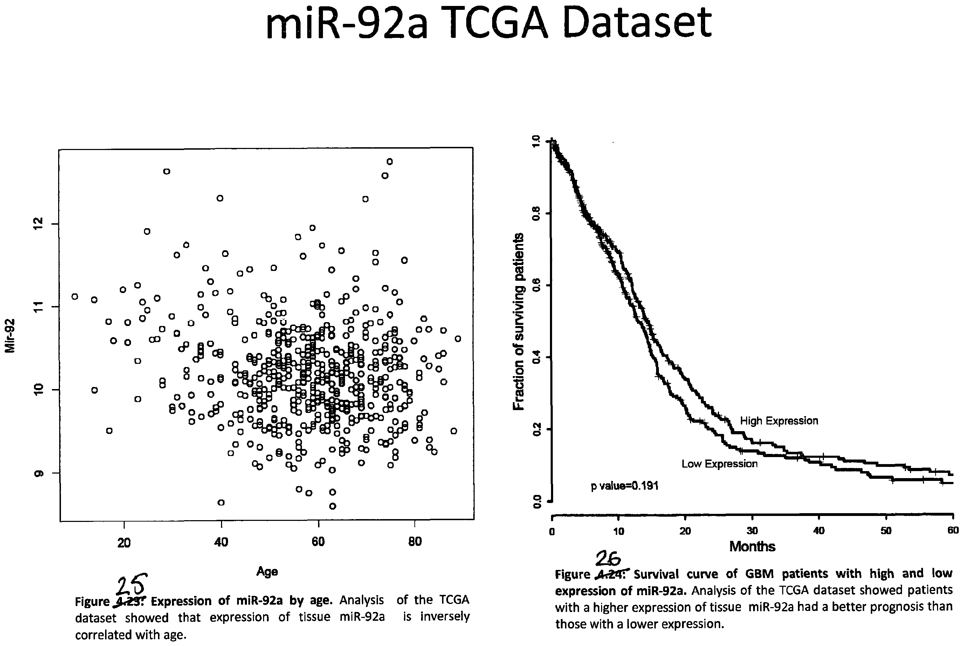

miRNA92a is also a promising prognostic biomarker. Initial studies from the 36 patients found that 92a was increased in serum samples from male GBM n=9 compared to male controls (n=9) (Figure 23) and also that the higher the expression, the better the prognosis in males (Figure 24). Power analysis showed that we needed to expand the patient cohort so we used another 72 patients. Analysis of TCGA database derived from glioblastoma tissue sample showed that from 558 patients there was not any difference in expression levels between sexes, but that once again high expression did correlate with better prognosis (P=0.001) (Figure 25 and 26).

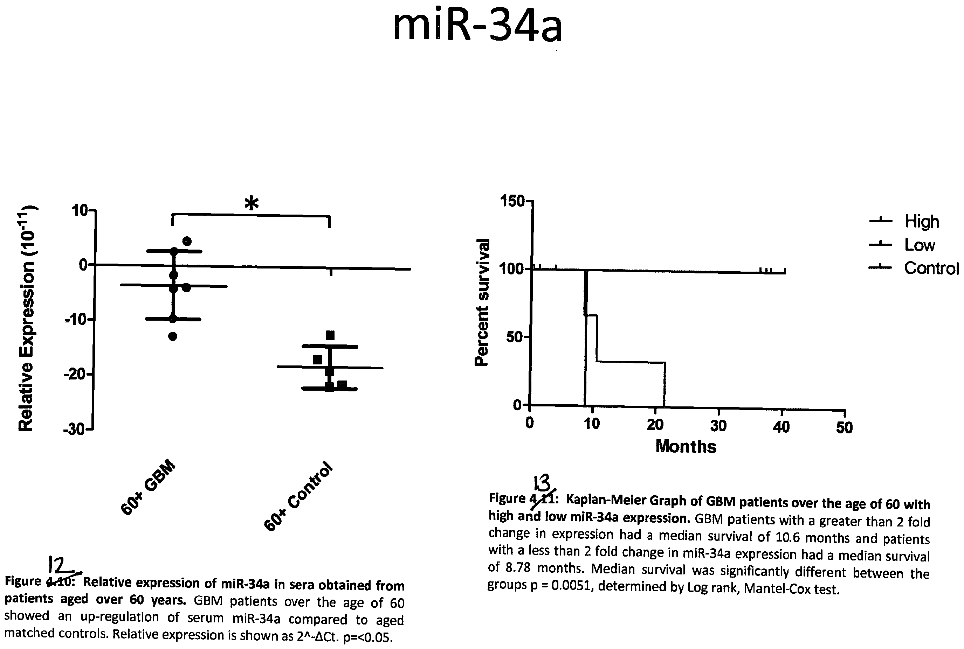

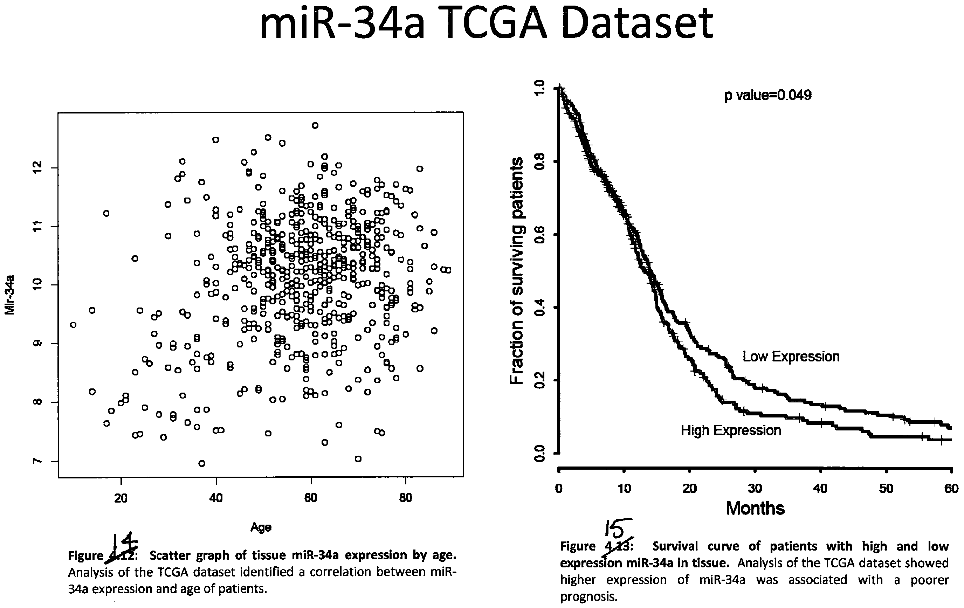

An additional finding was that microRNA-34a-5p (Hsa-miR-34a-5p) was found to be statistically significantly up-regulated in the serum of patients over the age of 60 when compared to age matched controls. This finding is surprising, since microRNA-34a-5p has previously been reported to be down-regulated in glioblastoma tissue. The present study represented the first occasion on which the abundance of this marker has been measured in the serum of glioblastoma patients. In serum samples from GBM patients the level of miRNA 34a correlated with increasing age. The results provided here show that miRNA34 is overexpressed in 60+ glioma patients and the higher the expression, the better the prognosis (Figure 12 and 13). In contrast dataset derived from cancer tissue samples showed that high levels of expression of miRNA34a in glioma tissues is associated with a poor prognosis (document and Figure 14 and 15). These data suggest that this miRNA does not have the same role(s) in different body tissue compartments.

### Sequence information

The present disclosure refers to various miRNAs using standard designations that will be recognised by those skilled in the art. For the avoidance of doubt, details of the sequences of various miRNAs referred to herein are set out below.

| **SEQ ID NO.** | **Name** | **Sequence** |
|---|---|---|
| 1 | hsa-miR-101-3p | uacaguacugugauaacugaa |
| 2 | hsa-miR-29b-3p | uagcaccauuugaaaucaguguu |
| 3 | hsa-miR-328 | |
| 4 | hsa-miR-9-5p | ucuuugguuaucuagcuguauga |
| 5 | Hsa-miR-106b-5p | uaaagugcugacagugcagau |
| 6 | Hsa-miR-107 | |
| 7 | Hsa-miR-125a-5p | ucccugagacccuuuaaccuguga |
| 8 | Hsa-miR-128 | |
| 9 | Hsa-miR-130b-3p | cagugcaaugaugaaagggcau |
| 10 | Hsa-miR-132-3p | uaacagucuacagccauggucg |
| 11 | Hsa-miR-138-5p | agcugguguugugaaucaggccg |
| 12 | Hsa-miR-141-3p | uaacacugucugguaaagaugg |
| 13 | Hsa-miR-146a-5p | ugagaacugaauuccauggguu |
| 14 | Hsa-miR-148a-3p | ucagugcacuacagaacuuugu |
| 15 | Hsa-miR-16-5p | uagcagcacguaaauauuggcg |
| 16 | Hsa-miR-17-5p | caaagugcuuacagugcagguag |
| 17 | Hsa-miR-17-3p | acugcagugaaggcacuuguag |
| 18 | Hsa-miR-182-5p | uuuggcaaugguagaacucacacu |
| 19 | Hsa-miR-183-5p | uauggcacugguagaauucacu |
| 20 | Hsa-miR-187-3p | ucgugucuuguguugcagccgg |
| 21 | Hsa-miR-18a-5p | uaaggugcaucuagugcagauag |
| 22 | Hsa-miR-190a | |
| 23 | Hsa-miR-19b-3p | ugugcaaauccaugcaaaacuga |
| 24 | Hsa-miR-200a-3p | uaacacugucugguaacgaugu |
| 25 | Hsa-miR-203a | |
| 26 | Hsa-miR-20a-5p | uaaagugcuuauagugcagguag |
| 27 | Hsa-miR-31-5p | aggcaagaugcuggcauagcu |
| 28 | Hsa-miR-326 | |
| 29 | Hsa-miR-425-5p | aaugacacgaucacucccguuga |
| 30 | Hsa-miR-7-5p | uggaagacuagugauuuuguugu |
| 31 | Hsa-miR-93-5p | caaagugcuguucgugcagguag |
| 32 | Hsa-miR-96-5p | uuuggcacuagcacauuuuugcu |
| 33 | Hsa-let-7b-5p | ugagguaguagguugugugguu |
| 34 | Hsa-miR-106b-5p | uaaagugcugacagugcagau |
| 35 | Hsa-miR-191-5p | caacggaaucccaaaagcagcug |
| 36 | Hsa-miR-24-3p | uggcucaguucagcaggaacag |
| 37 | Hsa-miR-25-3p | cauugcacuugucucggucuga |
| 38 | Hsa-miR-320a | |
| 39 | Hsa-miR-486-5p | uccuguacugagcugccccgag |
| 40 | Hsa-miR-451a | |
| 41 | Hsa-miR-92a-3p | uauugcacuugucccggccugu |
| 42 | Hsa-miR-34a-5p | UGGCAGUGUCUUAGCUGGUUGU |

## Claims

1. A method of predicting a clinical outcome in a patient with glioma, the method comprising:
assaying a serum sample from the patient for the presence of at least one miRNA selected from the group consisting of: Hsa-miR-20a-5p;
comparing the amount of the at least one miRNA present in the serum sample with a reference value;
wherein an increase in the amount of the at least one miRNA in the serum sample, such that the amount is more than twice the reference value, indicates a positive outcome in respect of the patient's glioma, wherein the reference value is the level of the same at least one miRNA present in a control subject without cancer.

2. A method according to claim 1, wherein a decrease in the amount of at the least one miRNA in the serum sample, as compared to twice the reference value, indicates a negative outcome in respect of the patient's glioma.

3. A method of monitoring the progression of glioma in a patient, the method comprising:
assaying a first serum sample from the patient, indicative of miRNA in the patient at a first timepoint, for the presence of at least one miRNA selected from the group consisting of: Hsa-miR-20a-5p to determine a first value for the abundance of the at least one miRNA present in the serum sample;
and assaying a second serum sample from the patient, indicative of miRNA in the patient at a second timepoint, for the presence of the same at least one miRNA to determine a second value for the abundance of the at least one miRNA present in the second serum sample; and
comparing the first and second values for the abundance of the at least one miRNA;
wherein an increase in the amount of the at least one miRNA present in the second serum sample as compared to the first serum sample, such that the amount is more than twice level of the same at least one miRNA present in a control subject without cancer, indicates an improvement in respect of the patient's glioma..

4. A method according to claim 3, wherein a decrease in the amount of the at least one miRNA present in the second serum sample as compared to the first serum sample, such that the amount is less than twice level of the same at least one miRNA present in a control subject without cancer, indicates worsening in respect of the patient's glioma.

5. A method of detecting glioma in a subject, the method comprising:
assaying a serum sample from the subject to determine the amount of at least one miRNA, selected from the group consisting of: Hsa-miR-20a-5p present in the serum sample;
comparing the amount of the at least one miRNA present in the serum sample with a reference value reflecting the level of the same at least one miRNA present in a control subject without cancer;
wherein an increase in the amount of at the least one miRNA in the serum sample, as compared to the reference value, indicates that the subject has glioma, and wherein if the increase is such that the amount of at the least one miRNA in the serum sample is more than twice the reference value, this indicates a positive outcome in respect of the patient's glioma.

6. A method according to any preceding claim, wherein the assay used to determine the presence or amount of the at least one miRNA comprises an amplification step in which miRNA in the patient sample is used as a template for the generation of artificial nucleic acid molecules.

## Patentansprüche

1. Verfahren zur Prognostizierung des klinischen Ergebnisses bei einem Patienten mit einem Gliom, wobei das Verfahren folgendes umfasst:
Untersuchen einer Serumprobe des Patienten auf die Präsenz mindestens einer miRNA, die ausgewählt ist aus der Gruppe bestehend aus: Hsa-miR-20a-5p;
Vergleichen der in der Serumprobe enthaltenen Menge der mindestens einen miRNA mit einem Referenzwert;
wobei ein derartiger Anstieg der Menge der mindestens einen miRNA in der Serumprobe, dass die Menge mehr als doppelt so groß ist wie der Referenzwert, ein positives Ergebnis in Bezug auf das Gliom des Patienten anzeigt, wobei der Referenzwert der Wert der gleichen mindestens einen miRNA ist, die in einer Kontrollperson ohne Krebs vorhanden ist.

2. Verfahren nach Anspruch 1, wobei ein Rückgang der Menge der mindestens einen miRNA in der Serumprobe im Vergleich zu dem zweifachen Wert des Referenzwerts ein negatives Ergebnis in Bezug auf das Gliom des Patienten anzeigt.

3. Verfahren zur Überwachung des Fortschreitens eines Glioms in einem Patienten, wobei das Verfahren folgendes umfasst:
Untersuchen einer ersten Serumprobe des Patienten, welche die miRNA in dem Patienten zu einem ersten Zeitpunkt anzeigt, auf die Präsenz mindestens einer miRNA, die ausgewählt ist aus der Gruppe bestehend aus: Hsa-miR-20a-5p, um einen ersten Wert für die Abundanz der in der Serumprobe vorhandenen mindestens einen miRNA zu bestimmen;
und Untersuchen einer zweiten Serumprobe des Patienten, welche die miRNA in dem Patienten zu einem zweiten Zeitpunkt anzeigt, auf die Präsenz der gleichen mindestens einen miRNA, um einen zweiten Wert für die Abundanz der in der zweiten Serumprobe vorhanden mindestens einen miRNA zu bestimmen; und
Vergleichen der ersten und zweiten Werte für die Abundanz der mindestens einen miRNA;
wobei ein derartiger Anstieg der Menge der in der zweiten Serumprobe vorhandenen mindestens einen miRNA im Vergleich zu der ersten Serumprobe, dass die Menge mehr als doppelt so groß ist wie der Wert der gleichen mindestens einen miRNA, die in einer Kontrollperson ohne Krebs vorhanden ist, eine Verbesserung in Bezug auf das Gliom des Patienten anzeigt.

4. Verfahren nach Anspruch 3, wobei ein derartiger Rückgang der in der zweiten Serumprobe vorhandenen mindestens einen miRNA im Vergleich zu der ersten Serumprobe, dass die Menge kleiner ist als der zweifache Wert der gleichen mindestens einen miRNA, die in einer Kontrollperson ohne Krebs vorhanden ist, eine Verschlechterung in Bezug auf das Gliom des Patienten anzeigt.

5. Verfahren für den Nachweis eines Glioms in einem Subjekt, wobei das Verfahren folgendes umfasst:
Untersuchen einer Serumprobe des Subjekts zur Bestimmung der in der Serumprobe vorhandenen Menge mindestens einer miRNA, die ausgewählt ist aus der Gruppe bestehend aus: Hsa-miR-20a-5p;
Vergleichen der in der Serumprobe enthaltenen Menge der mindestens einen miRNA mit einem Referenzwert, der den Wert der gleichen mindestens einen miRNA darstellt, die in einer Kontrollperson ohne Krebs vorhanden ist;
wobei ein Anstieg der Menge der mindestens einen miRNA in der Serumprobe im Vergleich zu dem Referenzwert anzeigt, dass das Subjekt ein Gliom aufweist, und wobei, wenn der Anstieg derart ist, dass die Menge der mindestens einen miRNA in der Serumprobe mehr als doppelt so groß ist wie der Referenzwert, dies ein positives Ergebnis in Bezug auf das Gliom des Patienten anzeigt.

6. Verfahren nach einem der vorstehenden Ansprüche, wobei die zur Bestimmung der Präsenz oder der Menge der mindestens einen miRNA verwendete Untersuchung einen Amplifikationsschritt umfasst, in welchem miRNA in der Patientenprobe als eine Vorlage für die Erzeugung künstlicher Nukleinsäuremoleküle verwendet wird.

## Revendications

1. Procédé de prédiction d'un résultat clinique chez un patient atteint d'un gliome, le procédé comprenant :
l'analyse d'un échantillon de sérum du patient en ce qui concerne la présence d'au moins un miARN sélectionné dans le groupe constitué par : Hsa-miR-20a-5p ;
la comparaison de la quantité de l'au moins un miARN présent dans l'échantillon de sérum avec une valeur de référence ;
dans lequel une augmentation de la quantité de l'au moins un miARN dans l'échantillon de sérum, telle que la quantité soit de plus du double de la valeur de référence, indique un résultat positif au regard du gliome du patient, la valeur de référence étant le niveau du même au moins un miARN présent dans un sujet témoin sans cancer.

2. Procédé selon la revendication 1, dans lequel une diminution de la quantité d'au moins un miARN dans l'échantillon de sérum, en comparaison du double de la valeur de référence, indique un résultat négatif au regard du gliome du patient.

3. Procédé de surveillance de la progression d'un gliome chez un patient, le procédé comprenant :
l'analyse d'un premier échantillon de sérum du patient, indicatif de miARN dans le patient à un premier point temporel, en ce qui concerne la présence d'au moins un miARN sélectionné dans le groupe constitué par : Hsa-miR-20a-5p afin de déterminer une première valeur pour l'abondance de l'au moins un miARN présent dans l'échantillon de sérum ;
et l'analyse d'un deuxième échantillon de sérum du patient, indicatif de miARN dans le patient à un deuxième point temporel, en ce qui concerne la présence du même au moins un miARN afin de déterminer une deuxième valeur pour l'abondance de l'au moins un miARN présent dans le deuxième échantillon de sérum ; et
la comparaison des première et deuxième valeurs pour l'abondance de l'au moins un miARN ;
dans lequel une augmentation de la quantité de l'au moins un miARN présent dans le deuxième échantillon de sérum en comparaison du premier échantillon de sérum, telle que la quantité soit de plus du double du niveau du même au moins un miARN présent dans un sujet témoin sans cancer, indique une amélioration au regard du gliome du patient.

4. Procédé selon la revendication 3, dans lequel une diminution de la quantité de l'au moins un miARN présent dans le deuxième échantillon de sérum en comparaison du premier échantillon de sérum, telle que la quantité soit de moins du double du niveau du même au moins un miARN présent dans un sujet témoin sans cancer, indique une aggravation au regard du gliome du patient.

5. Procédé de détection d'un gliome chez un sujet, le procédé comprenant :
l'analyse d'un échantillon de sérum du sujet afin de déterminer la quantité d'au moins un miARN, sélectionné dans le groupe constitué par : Hsa-miR-20a-5p présent dans l'échantillon de sérum ;
la comparaison de la quantité de l'au moins un miARN présent dans l'échantillon de sérum avec une valeur de référence reflétant le niveau du même au moins un miARN présent dans un sujet témoin sans cancer ;
dans lequel une augmentation de la quantité d'au moins un miARN dans l'échantillon de sérum, en comparaison de la valeur de référence, indique que le sujet a un gliome, et dans lequel si l'augmentation est telle que la quantité d'au moins un miARN dans l'échantillon de sérum soit de plus du double de la valeur de référence, ceci indique un résultat positif au regard du gliome du patient.

6. Procédé selon l'une quelconque des revendications précédentes, dans lequel l'analyse utilisée pour déterminer la présence ou quantité de l'au moins un miARN comprend une étape d'amplification dans laquelle un miARN dans l'échantillon du patient est utilisé en tant que modèle pour la génération de molécules d'acide nucléique artificielles.


### SEQUENCE LISTING

<110> The University of Central Lancashire
<120> DETECTION OF BRAIN CANCER
<130> P209778WO
<140> PCT/GB2014/000280
   <141> 2014-07-09
<150> GB1312336.9
   <151> 2013-07-09
<150> GB1400436.0
   <151> 2014-01-10
<160> 41
<170> PatentIn version 3.5
<210> 1
   <211> 21
   <212> RNA
   <213> Homo sapiens
<400> 1
   uacaguacug ugauaacuga a 21
<210> 2
   <211> 23
   <212> RNA
   <213> Homo sapiens
<400> 2
   uagcaccauu ugaaaucagu guu 23
<210> 3
   <211> 75
   <212> RNA
   <213> Homo sapiens
<400> 3
   uggagugggg gggcaggagg ggcucaggga gaaagugcau acagccccug gcccucucug 60
   cccuuccguc cccug 75
<210> 4
   <211> 23
   <212> RNA
   <213> Homo sapiens
<400> 4
   ucuuugguua ucuagcugua uga 23
<210> 5
   <211> 21
   <212> RNA
   <213> Homo sapiens
<400> 5
   uaaagugcug acagugcaga u 21
<210> 6
   <211> 81
   <212> RNA
   <213> Homo sapiens
<400> 6
   cucucugcuu ucagcuucuu uacaguguug ccuuguggca uggaguucaa gcagcauugu 60
   acagggcuau caaagcacag a 81
<210> 7
   <211> 24
   <212> RNA
   <213> Homo sapiens
<400> 7
   ucccugagac ccuuuaaccu guga 24
<210> 8
   <211> 0
   <212> RNA
   <213> Homo sapiens
<400> 8 00
<210> 9
   <211> 22
   <212> RNA
   <213> Homo sapiens
<400> 9
   cagugcaaug augaaagggc au 22
<210> 10
   <211> 22
   <212> RNA
   <213> Homo sapiens
<400> 10
   uaacagucua cagccauggu cg 22
<210> 11
   <211> 23
   <212> RNA
   <213> Homo sapiens
<400> 11
   agcugguguu gugaaucagg ccg 23
<210> 12
   <211> 22
   <212> RNA
   <213> Homo sapiens
<400> 12
   uaacacuguc ugguaaagau gg 22
<210> 13
   <211> 22
   <212> RNA
   <213> Homo sapiens
<400> 13
   ugagaacuga auuccauggg uu 22
<210> 14
   <211> 22
   <212> RNA
   <213> Homo sapiens
<400> 14
   ucagugcacu acagaacuuu gu 22
<210> 15
   <211> 22
   <212> RNA
   <213> Homo sapiens
<400> 15
   uagcagcacg uaaauauugg cg 22
<210> 16
   <211> 23
   <212> RNA
   <213> Homo sapiens
<400> 16
   caaagugcuu acagugcagg uag 23
<210> 17
   <211> 22
   <212> RNA
   <213> Homo sapiens
<400> 17
   acugcaguga aggcacuugu ag 22
<210> 18
   <211> 24
   <212> RNA
   <213> Homo sapiens
<400> 18
   uuuggcaaug guagaacuca cacu 24
<210> 19
   <211> 22
   <212> RNA
   <213> Homo sapiens
<400> 19
   uauggcacug guagaauuca cu 22
<210> 20
   <211> 22
   <212> RNA
   <213> Homo sapiens
<400> 20
   ucgugucuug uguugcagcc gg 22
<210> 21
   <211> 23
   <212> RNA
   <213> Homo sapiens
<400> 21
   uaaggugcau cuagugcaga uag 23
<210> 22
   <211> 85
   <212> RNA
   <213> Homo sapiens
<400> 22
   ugcaggccuc ugugugauau guuugauaua uuagguuguu auuuaaucca acuauauauc 60
   aaacauauuc cuacaguguc uugcc 85
<210> 23
   <211> 23
   <212> RNA
   <213> Homo sapiens
<400> 23
   ugugcaaauc caugcaaaac uga 23
<210> 24
   <211> 22
   <212> RNA
   <213> Homo sapiens
<400> 24
   uaacacuguc ugguaacgau gu 22
<210> 25
   <211> 110
   <212> RNA
   <213> Homo sapiens
<400> 25
   guguugggga cucgcgcgcu ggguccagug guucuuaaca guucaacagu ucuguagcgc 60
   aauugugaaa uguuuaggac cacuagaccc ggcgggcgcg gcgacagcga 110
<210> 26
   <211> 23
   <212> RNA
   <213> Homo sapiens
<400> 26
   uaaagugcuu auagugcagg uag 23
<210> 27
   <211> 21
   <212> RNA
   <213> Homo sapiens
<400> 27
   aggcaagaug cuggcauagc u 21
<210> 28
   <211> 95
   <212> RNA
   <213> Homo sapiens
<400> 28
   cucaucuguc uguugggcug gaggcagggc cuuugugaag gcggguggug cucagaucgc 60
   cucugggccc uuccuccagc cccgaggcgg auuca 95
<210> 29
   <211> 23
   <212> RNA
   <213> Homo sapiens
<400> 29
   aaugacacga ucacucccgu uga 23
<210> 30
   <211> 23
   <212> RNA
   <213> Homo sapiens
<400> 30
   uggaagacua gugauuuugu ugu 23
<210> 31
   <211> 23
   <212> RNA
   <213> Homo sapiens
<400> 31
   caaagugcug uucgugcagg uag 23
<210> 32
   <211> 23
   <212> RNA
   <213> Homo sapiens
<400> 32
   uuuggcacua gcacauuuuu gcu 23
<210> 33
   <211> 22
   <212> RNA
   <213> Homo sapiens
<400> 33
   ugagguagua gguugugugg uu 22
<210> 34
   <211> 21
   <212> RNA
   <213> Homo sapiens
<400> 34
   uaaagugcug acagugcaga u 21
<210> 35
   <211> 23
   <212> RNA
   <213> Homo sapiens
<400> 35
   caacggaauc ccaaaagcag cug 23
<210> 36
   <211> 22
   <212> RNA
   <213> Homo sapiens
<400> 36
   uggcucaguu cagcaggaac ag 22
<210> 37
   <211> 22
   <212> RNA
   <213> Homo sapiens
<400> 37
   cauugcacuu gucucggucu ga 22
<210> 38
   <211> 82
   <212> RNA
   <213> Homo sapiens
<400> 38
   gcuucgcucc ccuccgccuu cucuucccgg uucuucccgg agucgggaaa agcuggguug 60
   agagggcgaa aaaggaugag gu 82
<210> 39
   <211> 22
   <212> RNA
   <213> Homo sapiens
<400> 39
   uccuguacug agcugccccg ag 22
<210> 40
   <211> 72
   <212> RNA
   <213> Homo sapiens
<400> 40
   cuugggaaug gcaaggaaac cguuaccauu acugaguuua guaaugguaa ugguucucuu 60
   gcuauaccca ga 72
<210> 41
   <211> 22
   <212> RNA
   <213> Homo sapiens
<400> 41
   uauugcacuu gucccggccu gu 22
<210> 42
   <211> 22
   <212> RNA
   <213> Homo sapiens
<400> 42
   uggcaguguc uuagcugguu gu 22